# EUROPEAN PATENT APPLICATION

(11) **EP 2 133 321 A1**
(43) Date of publication of application: **16.12.2009**
(21) Application number: 08721388.0
(22) Date of filing: 05.03.2008
(51) Int. Cl.: C07C 25/22, C07C 49/813, C08G 61/12, G01N 21/27, H01L 31/04

(54) **FLUORINE-CONTAINING POLYCYCLIC AROMATIC COMPOUND, FLUORINE-CONTAINING POLYMER, ORGANIC THIN FILM AND ORGANIC THIN FILM DEVICE**

(30) Priority: 09.03.2007 JP 2007060699
(71) Applicant: Sumitomo Chemical Company, Limited, Tokyo 104-8260 (JP)
(72) Inventor: IE, Yutaka, Suita-shi Osaka 565-0871 (JP); ASO, Yoshio, Suita-shi Osaka 565-0871 (JP); NITANI, Masashi, Suita-shi Osaka 565-0871 (JP); UEDA, Masato, Tsukuba-shi Ibaraki 305-0046 (JP)
(74) Representative: Duckworth, Timothy John
(86) International application number: PCT/JP2008/053968
(87) International publication number: WO 2008/111461

(57) **Abstract**

A fluorine-containing polycyclic aromatic compound represented by the following formula (I) is provided. According to present invention, a novel compound capable of being utilized as an organic n-type semiconductor having an excellent electron transport property is provided, 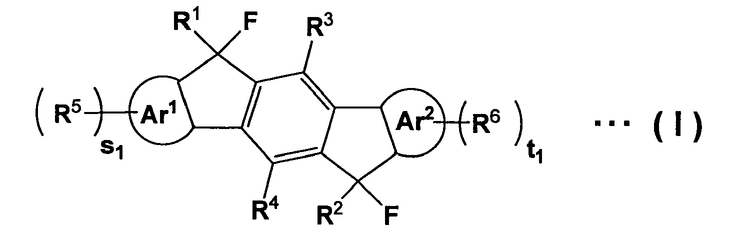 wherein in formula (I), Ar¹ and Ar² each independently represent an aromatic hydrocarbon group having at least six carbon atoms or a heterocyclic group having at least four carbon atoms; R¹, R², R³ and R⁴ each independently represent a hydrogen atom, a halogen atom, or a monovalent group; R⁵ and R⁶ each independently represent a hydrogen atom or a monovalent organic group that may be substituted with a substituent; s₁ and t₁ each independently represent an integer of at least 2; with the proviso that at least one of R⁵ and R⁶ is the abovementioned monovalent organic group where at least one of all the hydrogen atoms of the monovalent organic group including the substituent is substituted with a fluorine atom.

## Description

### Technical Field

The present invention relates to fluorine-containing polycyclic aromatic compound, fluorine-containing polymer, organic thin film and organic thin film device.

### Background Art

Although a thin film containing an organic material having an electron transport property or a hole transport property is expected to be applied towards organic thin film devices such as organic thin film transistors, organic solar cells and photosensors, because of the difficulty in obtaining an organic n-type semiconductor (demonstrating an electron transport property) when compared with an organic p-type semiconductor (demonstrating a hole transport property), various studies have been conducted towards the development of an organic n-type semiconductor.

A fluoroalkyl group introduced π-conjugated compound is a compound which could potentially be developed into a material with electron transport properties such as organic n-type semiconductors, in order to increase an electron accepting property. Accordingly, there have been extensive studies conducted in recent years on compounds containing a fluoroalkyl group introduced into a thiophene ring (Patent Documents 1 to 4).

On the other hand, various ladder-type fluorenes or polythiophenes having a cross-linked structure have been studied in order to improve a planarity of a molecular structure (Non-Patent Document 1 and 2, and Patent Document 5).

Moreover, a tetrafluoropentacene and the like, in which a fluorinated pentacene with a high degree of planarity, was studied as an organic n-type semiconductor (Patent Document 6).
Patent Document 1: U.S. Patent Application Publication No. 2004/186266;
Patent Document 2: U.S. Patent Application Publication No. 2004/183068;
Patent Document 3: WO 2003/010778;
Patent Document 4: European Patent Application Publication No. 1279689;
Patent Document 5: Japanese Patent Application Laid-Open Publication No. 2004-339516;
Patent Document 6: Japanese Patent Application Laid-Open Publication No. 2005-235923;
Non-Patent Document 1: Paolo Coppo et al., J. Mat. Commun. 2002, 12(9), 2597; and
Non-Patent Document 2: Jacob, Josemon et al., J. American Chem. Soc. 2004, 126(22), 6987.

### Disclosure of the Invention

### Problem to be Solved by the Invention

However, it is difficult to say whether a known material such as that described above is sufficient in capability as an organic n-type semiconductor, and further there is a need for an organic n-type semiconductor having an improved electron transport property.

Accordingly, the objective of the present invention is to provide a novel polymer and novel compound capable of being utilized as an organic n-type semiconductor with an excellent electron transport property. A further objective of the present invention is to provide an organic thin film containing this novel compound and/or novel polymer, as well as an organic thin film device including this organic thin film.

### Means for Solving the Problem

In order to achieve the abovementioned objective, the present invention provides a fluorine-containing polycyclic aromatic compound represented by the following formula (I). 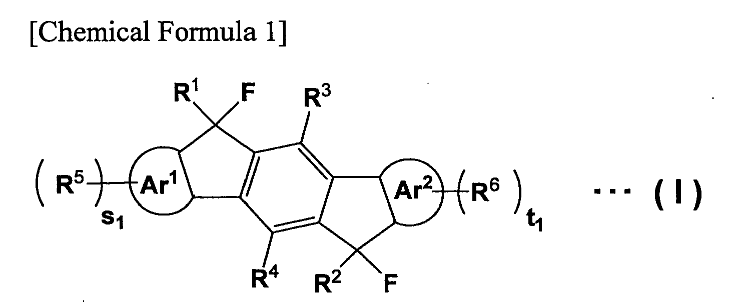 In the formula (I), Ar¹ and Ar² each independently represent an aromatic hydrocarbon group having at least six carbon atoms or a heterocyclic group having at least four carbon atoms; R¹, R², R³ and R⁴ each independently represent a hydrogen atom, a halogen atom, or a monovalent group; R⁵ and R⁶ each independently represent a hydrogen atom or a monovalent organic group that may be substituted with a substituent, wherein the monovalent organic group is selected from among an alkyl group, an alkoxy group, an acyl group, an aryl group, an aryloxy group, an arylalkyl group, an arylalkoxy group and a heterocyclic group, and at least one of all the hydrogen atoms of the monovalent organic group including the substituent may be substituted with a fluorine atom; and s₁ and t₁ each independently represent an integer of least 2. However, at least one of R⁵ and R⁶ is the monovalent organic group, in which at least one of all the hydrogen atoms of the monovalent organic group including the substituent is substituted with a fluorine atom. Moreover, the plurality of R⁵ may be the same as or different from each other, and the plurality of R⁶ may be the same as or different from each other.
Furthermore, the Ar¹ that is an aromatic hydrocarbon group having at least six carbon atoms refers to the ring surrounding the symbol "Ar¹" in the formula (I) that is formed of an aromatic hydrocarbon group having a total of at least six carbon atoms (also similar to that of Ar²), and the Ar¹ that is a heterocyclic group having at least four carbon atoms refers to the ring surrounding the symbol "Ar¹" in the formula (I) that is formed of a heterocyclic group having a total of at least four carbon atoms (also similar to that of Ar²).
Moreover, the value of s₁ and t₁ may be thought of as follows. In cases where Ar¹ is an aromatic hydrocarbon having at least six carbon atoms, the total number of hydrogen atoms linked to carbons atoms in the above group is calculated when s₁ is assumed to be 0. If the value thereof is N1ₐ₁₁, s1= N1ₐ₁₁ (Ar¹ is also the same in cases where it is a heterocyclic group having at least four carbon atoms). Moreover, in cases where Ar² is an aromatic hydrocarbon having at least six carbon atoms, the total number of hydrogen atoms linked to carbon atoms in the above group is calculated when t₁ is assumed to be 0. If the value thereof is M1ₐ₁₁, t₁= M1ₐ₁₁ (Ar² is also the same in cases where it is a heterocyclic group having at least four carbon atoms). Accordingly, the values of s₁ and t₁ may be primarily determined according to the number hydrogen atoms linked to carbon atoms in which there are at least four carbon atoms or an aromatic hydrocarbon group of at least six carbons.

The present invention provides a fluorine-containing polymer having a repeating unit represented by the following formula (III). 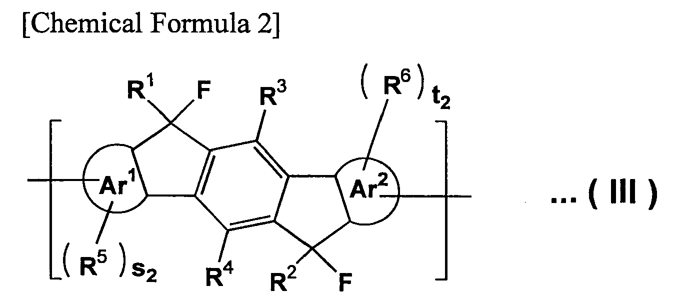 In the formula (III), Ar¹ and Ar² each independently represent an aromatic hydrocarbon group having at least six carbon atoms or a heterocyclic group having at least four carbon atoms; R¹, R², R³ and R⁴ each independently represent a hydrogen atom, a halogen atom, or a monovalent group; R⁵ and R⁶ each independently represent a hydrogen atom or a monovalent organic group that may be substituted with a substituent, wherein the monovalent organic group is selected from among an alkyl group, an alkoxy group, an acyl group, an aryl group, an aryloxy group, an arylalkyl group, an arylalkoxy group and a heterocyclic group, and at least one of all the hydrogen atoms of the monovalent organic group including the substituent may be substituted with a fluorine atom; and s₂ and t₂ each independently represent an integer of at least 1. Moreover, the plurality of R⁵ may be the same as or different from each other when s₂ is at least 2, and the plurality of R⁶ may be the same as or different from each other when t₂ is at least 2.
Moreover, the Ar¹ that is an aromatic hydrocarbon group having at least six carbon atoms refers to the ring surrounding the symbol "Ar¹" in the formula (III) that is formed of an aromatic hydrocarbon group having a total of at least six carbon atoms (also similar to that of Ar²), and the Ar¹ that is a heterocyclic group having at least four carbon atoms refers to the ring surrounding the symbol "Ar¹" in the formula (III) that is formed of a heterocyclic group having a total of at least four carbon atoms (similar to that of Ar²).
Moreover, the value of s₂ and t₂ may be thought of as follows. In cases where Ar¹ is an aromatic hydrocarbon having at least six carbon atoms, the total number of hydrogen atoms linked to carbon atoms in the above group is calculated when s₂ is assumed to be 0. If the value thereof is N2ₐ₁₁, s₂= N2ₐ₁₁ (Ar¹ is also the same in cases where it is a heterocyclic group having at least four carbon atoms). Moreover, in cases where Ar² is an aromatic hydrocarbon having at least six carbon atoms, the total number of hydrogen atoms linked to carbon atoms in the above group is calculated when t₂ is assumed to be 0. If the value thereof is M2ₐ₁₁, t₂= M2ₐ₁₁ (Ar² is also the same in cases where it is a heterocyclic group having at least four carbon atoms). Accordingly, the values of s₂ and t₂ may be primarily determined according to the number hydrogen atoms linked to carbon atoms in which there are at least four carbon atoms or an aromatic hydrocarbon group of at least six carbons.

A fluorine-containing polycyclic aromatic compound and fluorine-containing polymer providing such as skeleton has a π-conjugated planarity of both rings that is favorable, is capable of showing a sufficiently low LUMO via the introduction of a fluorine atom, and may be utilized as an organic n-type semiconductor having a superior electron transport property. Moreover, since the fluorine-containing polycyclic aromatic compound and fluorine-containing polymer is chemically stable and superior in solubility with respect to an organic solution, by using these to form a thin film, an organic film thin film device that is superior in performance can be manufactured.

The present invention further provides an organic thin film containing a fluorine-containing polycyclic aromatic compound and/or fluorine-containing polymer, and an organic thin film device including the abovementioned thin film.

Since the above organic thin film and organic thin film device contains the fluorine-containing polycyclic aromatic compound or fluorine-containing polymer of the present invention, it has sufficiently low LUMO, and demonstrates a superior electron transport property.

### Effects of the Invention

According to the present invention, a novel fluorine-containing polycyclic aromatic compound and novel fluorine-containing polymer capable of being utilized as an organic n-type semiconductor having a superior electron transport property may be provided. Moreover, an organic film containing the fluorine-containing polycyclic aromatic compound or the fluorine-containing polymer, and an organic thin film device including this organic thin film may be provided. Furthermore, since the fluorine-containing polycyclic aromatic compound and the fluorine containing polymer are superior in an electron transport property, an organic thin film transistor including the abovementioned organic thin film normally shows a favorable Id-Vg property, an organic thin film solar cell normally shows a superior voltage-current property, and a photosensor normally shows a favorable photo-to-dark-current ratio.

### Brief Description of the Drawings

FIG. 1 is a schematic cross-sectional diagram of an organic thin film transistor according to a first embodiment of the present invention.
FIG. 2 is a schematic cross-sectional diagram of an organic thin film transistor according to a second embodiment of the present invention.
FIG 3 is a schematic cross-sectional diagram of an organic thin film transistor according to a third embodiment of the present invention.
FIG. 4 is a schematic cross-sectional diagram of an organic thin film transistor according to a fourth embodiment of the present invention.
FIG. 5 is a schematic cross-sectional diagram of an organic thin film transistor according to a fifth embodiment of the present invention.
FIG 6 is a schematic cross-sectional diagram of an organic thin film transistor according to a sixth embodiment of the present invention.
FIG. 7 is a schematic cross-sectional diagram of an organic thin film transistor according to a seventh embodiment of the present invention.
FIG. 8 is a schematic cross-sectional diagram of a solar cell according to an embodiment.
FIG 9 is a schematic cross-sectional diagram of a photosensor according to a first embodiment.
FIG 10 is a schematic cross-sectional diagram of a photosensor according to a second embodiment.
FIG. 11 is a schematic cross-sectional diagram of a photosensor according to a third embodiment.
FIG 12 is a diagram showing a dihedral angle formed by a ring of a repeating unit represented by the general formula (TV) and a ring of a repeating unit represented by the general formula (VI).

### Explanation of Reference Numerals

1: Substrate; 2: Active layer; 2a: Active layer; 3: Insulating layer; 4: Gate electrode; 5: Source electrode; 6: Drain electrode; 7a: First electrode; 7b: Second electrode; 8: Charge generation layer; 100: Organic thin film transistor according to a first embodiment; 110: Organic thin film transistor according to a second embodiment; 120: Organic thin film transistor according to a third embodiment; 130: Organic thin film transistor according to a fourth embodiment; 140: Organic thin film transistor according to a fifth embodiment; 150: Organic thin film transistor according to a sixth embodiment; 160: Organic thin film transistor according to a seventh embodiment; 200: Solar cell according to an embodiment; 300: Photosensor according to a first embodiment; 310: Photosensor according to a second embodiment; 320: Photosensor according to a third embodiment.

### Best Modes for Carrying Out the Invention

Hereinafter, preferred embodiments of the present invention will be described in greater detail with reference to the drawings when necessary. Moreover, in cases where the same element is appended with the same reference numeral as that described in the drawings duplicate descriptions thereof will be omitted. Furthermore, positional relationships such as top/bottom or left/right relationships and the like, unless specifically indicated otherwise are based on the positional relationships indicated in the drawings. In addition, the dimensional proportions of the drawings are not specifically limited to the proportions of the diagrammatical representations thereof.

A fluorine-containing polycyclic aromatic compound of the present invention has a structure represented by the abovementioned general formula (I).

In the abovementioned general formula (I), Ar¹ and Ar² each independently represent an aromatic hydrocarbon group having at least six carbon atoms or a heterocyclic group having at least four carbon atoms; R¹, R², R³ and R⁴ each independently represent a hydrogen atom, a halogen atom, or a monovalent group; R⁵ and R⁶ each independently represent a hydrogen atom or a monovalent organic group, which may be substituted by one or a plurality of arbitrary substituents, and at least one of all the hydrogen atoms of the monovalent organic group including the substituent may be substituted with a fluorine atom. However, at least one of R⁵ and R⁶ is the abovementioned monovalent organic group, in which at least one of all the hydrogen atoms of the monovalent organic group including the substituent is substituted with a fluorine atom. Moreover, it is preferable that the organic group is an alkyl group, an alkoxy group, an acyl group, an aryl group, an aryloxy group, an arylalkyl group, an arylalkoxy group or a heterocyclic group. s₁ and t₁ each independently represent an integer of at least 2. Furthermore, although Ar¹ and Ar² may be the same as or different from each other, in order to facilitate the production thereof, it is preferable that Ar¹ and Ar² are the same. In addition, the plurality of R⁵ may be the same as or different from each other, and the plurality of R⁶ may be the same as or different from each other.

Moreover, the compound represented by the abovementioned general formula (I) is preferably the compound the represented by the below-mentioned general formula (II).

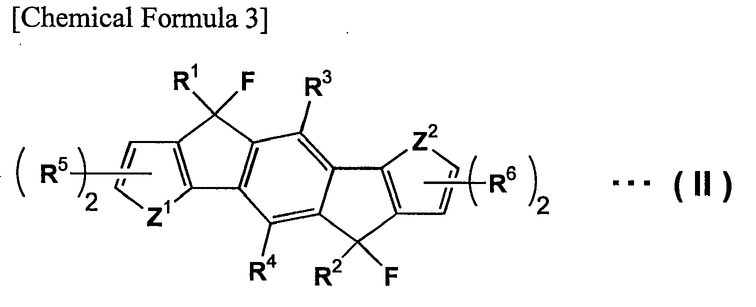

In the abovementioned general formula (II), R¹, R², R³, R⁴, R⁵, and R⁶ are the same as above in meaning, and Z¹ and Z² each independently represent any one of groups represented by the below-mentioned formulae (i) to (ix). However, at least one of R⁵ and R⁶ is the abovementioned monovalent organic group, in which at least one of all the hydrogen atoms of the monovalent organic group including the substituent is substituted with a fluorine atom. Furthermore, R⁷, R⁸, R⁹ and R¹⁰ each independently represent a hydrogen atom, a halogen atom, or a monovalent group, wherein R⁷ and R⁸ may be linked to each other to form a ring. In addition, the group represented by the below-mentioned formula (ii) may be reversed from left to right.

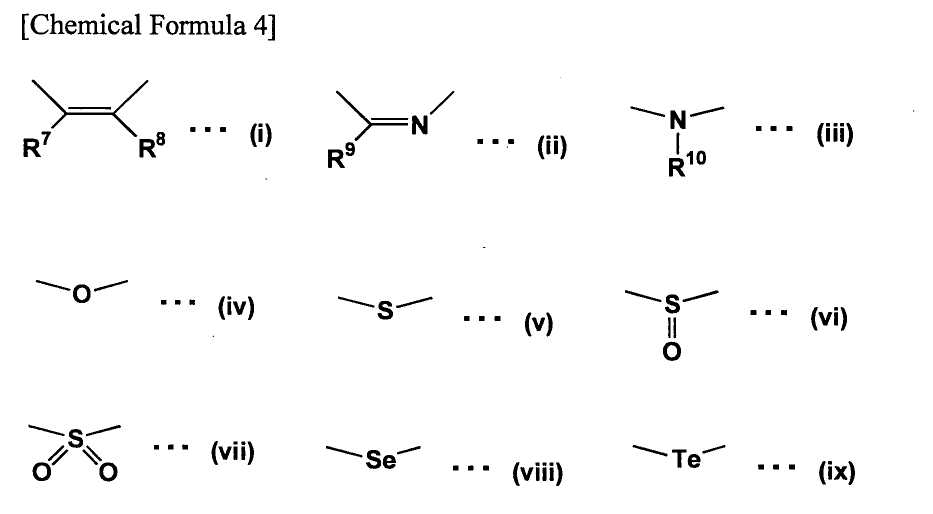

Moreover, the fluorine-containing polymer of the present invention includes a repeating unit represented by the abovementioned general formula (III). Specifically, the fluorine-containing polymer of the present invention includes at least one, and more preferably at least two, of the repeating unit represented by the abovementioned general formula (III) (for example, as the repeating unit represented by the general formula (IV) hereinafter), or it may include another repeating unit. The plurality of R¹, R², R³ and R⁴ in the fluorine-containing polymer may each be the same or different. Furthermore, in order facilitate the production thereof it is preferable that the plurality of R¹, R², R³, and R⁴ present are each respectively the same.

In the formula (III), Ar¹ and Ar² each independently represent an aromatic hydrocarbon group having at least six carbon atoms or a heterocyclic group having at least four carbon atoms, which may be substituted by one or a plurality of arbitrary a substituents; and R¹, R², R³ and R⁴ each independently represent a hydrogen atom, a halogen atom, or a monovalent group. R⁵ and R⁶ each independently represent a hydrogen atom or a monovalent organic group, where the organic group may be substituted by one or a plurality of arbitrary substituents, and at least one of all the hydrogen atoms of the monovalent organic group including the substituent may be substituted with a fluorine atom. Moreover, it is preferable that the organic group is an alkyl group, an alkoxy group, an acyl group, an aryl group, an aryloxy group, an arylalkyl group, an arylalkoxy group or a heterocyclic group. s₂ and t₂ each independently represent an integer of at least 1. Furthermore, although Ar¹ and Ar² may be the same as or different from each other, in order to facilitate the production thereof, it is preferable that Ar¹ and Ar² are the same. In addition, the plurality of R⁵ may be the same as or different from each other when s₂ is at least 2, and the plurality of R⁶ may be the same as or different from each other when t₂ is at least 2.

It is preferable that the repeating unit represented by the abovementioned general formula (III) is the repeating unit represented by the below-mentioned general formula (IV). Including a repeating unit such as this allows for utilization as an organic n-type semiconductor that is particularly superior in its electron transport property.

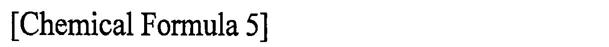

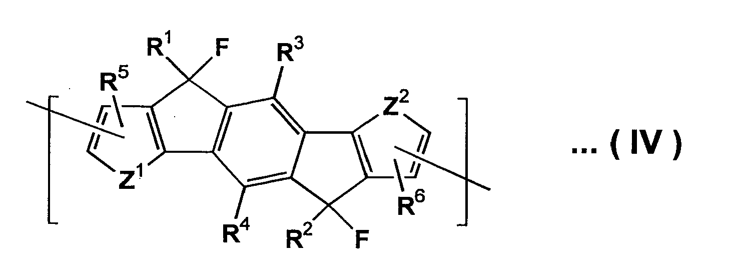

In the abovementioned general formula (IV), R¹, R², R³, R⁴, R⁵ and R⁶ are the same as above in meaning, and Z¹ and Z² each independently represent any one of groups represented by the below-mentioned formulae (i) to (ix). Furthermore, R⁷, R⁸, R⁹ and R¹⁰ each independently represent a hydrogen atom, a halogen atom, or a monovalent group, wherein R⁷ and R⁸ may be linked to each other to form a ring. In addition, the group represented by the above-mentioned formula (ii) may be reversed from left to right.

Although the fluorine-containing polymer of the present invention may include one type of repeating unit or may include a plurality of different types of repeating units as the repeating unit represented by the abovementioned general formula (III) in the molecule, it is preferable that one type of repeating unit is included when considering the facilitation of production.

The fluorine-containing polymer of the present invention preferably includes at least one repeating unit represented by the abovementioned general formula (III) and at least one repeating unit represented by the below-mentioned general formula (V) that differs from the repeating unit represent by the abovementioned general formula (III), and more preferably includes at least one repeating unit represented by the abovementioned general formula (III) and at least one repeating unit represented by the below-mentioned general formula (VI). Having such a structure will widen the range of allowable changes in the solubility, the mechanical properties, the thermal properties, or the electronic properties. Moreover, Ar³ in the below-mentioned general formula (V) refers to a divalent aromatic hydrocarbon group or a divalent heterocyclic group (these groups may also be substituted by a substituent). The ratio between the repeating unit represented by the general formula (III) (preferably the repeating unit represented by the abovementioned general formula (IV) and the repeating unit represented by the general formula (V) (preferably the repeating unit represented by the below-mentioned general formula (VI)), is preferably 10 mol to 1000 mol of the latter to 100 mol of the former, more preferably 25 mol to 400 mol of the latter to 100 mol of the former, and even more preferably 50 mol to 200 mol of the latter to 100 mol of the former.

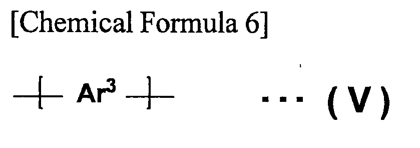

In the present case, it is preferred that Ar³ is a group represented by the below-mentioned general formula (VI). Including a repeating unit such as this allows for the solubility and electronic properties to be easily controlled. In the formula Z³ may be the same or different as Z¹ or Z², and represents any one of groups represented by the abovementioned formulae (i) to (ix). Furthermore, R¹¹ and R¹² each independently represent a hydrogen atom, a halogen atom, or a monovalent group, wherein R¹¹ and R¹² may be linked to each other to form a ring. However, R⁷, R⁸, R⁹, and R¹⁰ are the same as above in meaning.

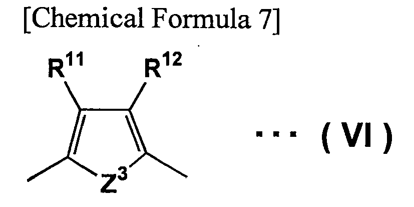

In the general formula (I), the aromatic hydrocarbon group with at least six carbon atoms that is represented by Ar¹ refers to the remaining atomic grouping after (2+s₁) hydrogen atoms are excluded from a fused ring or benzene ring having at least six carbon atoms, and the aromatic hydrocarbon group with at least six carbon atoms that is represented by Ar² refers to the remaining atomic grouping after (2+t₁) hydrogen atoms are excluded from a fused ring or benzene ring having at least six carbon atoms. Moreover, in the general formula (III), the aromatic hydrocarbon group with at least six carbon atoms that is represented by Ar¹ refers to the remaining atomic grouping after (3+s₂) hydrogen atoms are excluded from a fused ring or benzene ring having at least six carbon atoms, and the aromatic hydrocarbon group with at least six carbon atoms that is represented by Ar² refers to the remaining atomic grouping after (3+t₂) hydrogen atoms are excluded from a fused ring or benzene ring having at least six carbon atoms. The carbon number of the above-described group is normally 6 to 60, and preferably 6 to 20. As the fused ring, a naphthalene ring, an anthracene ring, a pyrene ring, a perylene ring, and fluorene may be exemplified. Furthermore, the carbon number of the aromatic hydrocarbon group may include the carbon number of the substituent. In such a case, the aromatic hydrocarbon group does not include the carbon number of the substituent. In addition, as the substituent, a halogen atom, a saturated or unsaturated hydrocarbon group, aryl group, alkoxy group, aryloxy group, a monovalent heterocyclic group, an amino group, a nitro group, and a cyano group may be exemplified.

In the general formula (I), the heterocyclic group with at least four carbon atoms that is represented by Ar¹ refers to the remaining atomic grouping after (2+s₁) hydrogen atoms are excluded from a heterocyclic compound, and the heterocyclic group with at least four carbon atoms that is represented by Ar² refers to the remaining atomic grouping after (2+t₁) hydrogen atoms are excluded from a heterocyclic compound. Moreover, in the general formula (III), the heterocyclic group with at least four carbon atoms that is represented by Ar¹ refers to the remaining atomic grouping after (3+s₂) hydrogen atoms are excluded from a heterocyclic compound, and the heterocyclic group with at least four carbon atoms that is represented by Ar² refers to the remaining atomic grouping after (3+t₂) hydrogen atoms are excluded from a heterocyclic compound. The carbon number of the above-described group is normally 4 to 60, and preferably 4 to 20. Furthermore, a substituent may be included in the heterocyclic group, and the carbon number of the substituent is not included in the carbon number of the heterocyclic group. In addition, as the substituent, a halogen atom, a saturated or unsaturated hydrocarbon group, an aryl group, an alkoxy group, an aryloxy group, a monovalent heterocyclic group, an amino group, a nitro group, and a cyano group may be exemplified.

The divalent aromatic hydrocarbon group that is represented by Ar³ refers to the remaining atomic grouping after two hydrogen atoms are excluded from a fused ring or benzene ring having at least six carbon atoms, which normally has a carbon number of 6 to 60, and preferably 6 to 20. As the fused ring, a naphthalene ring, an anthracene ring, a tetracene ring, a pentacene ring, a pyrene ring, a perylene ring, and a fluorene ring may be exemplified. Among these a remaining atomic grouping after two hydrogen atoms are excluded from a fluorene ring or benzene ring is especially preferable. Moreover, the aromatic hydrocarbon group may include a substituent. In such a case, the carbon number of the substituent is not included in the carbon number of the divalent aromatic hydrocarbon group. Furthermore, as the substituent, a halogen atom, a saturated or unsaturated hydrocarbon group, an aryl group, an alkoxy group, an aryloxy group, a monovalent heterocyclic group, an amino group, a nitro group, and a cyano group may be exemplified.

In addition, the divalent aromatic hydrocarbon group that is represented by Ar³ refers to the remaining atomic grouping after two hydrogen atoms are excluded from a heterocyclic compound, which normally has a carbon number of 4 to 60, and preferably 4 to 20. As the abovementioned heterocyclic compound, a compound in which two to six thiophene rings such as a thiophene ring, thienothiophene ring and dithienothiophene ring are fused; a thiazole ring; a pyrrole ring; a pryidine ring; a pyrimidine ring; a pyrazine ring; and a triazine ring may be exemplified, with a compound in which 2 to 6 thiophene rings such as a thiophene ring, a thienothiophene ring and dithienothiophene ring are fused rings being preferable. Furthermore, a substituent may be included in the heterocyclic group, and the carbon number of the substituent is not included in the carbon number of the divalent heterocyclic group. In addition, as the substituent, a halogen atom, a saturated or unsaturated hydrocarbon group, an aryl group, an alkoxy group, an aryloxy group, a monovalent heterocyclic group, an amino group, a nitro group, and a cyano group may be exemplified.

Thus, the heterocyclic compound referred to here is an organic compound with a ring structure in which the elements composing the ring include not only carbon but also heteroatoms such as oxygen, sulfur, nitrogen, phosphorus, boron, and silicon.

Z¹ and Z² in the general formulae (II) and (IV), for example, may be preferably represented by any one of groups represented by the abovementioned formulae (i) to (v), more preferably represented by any of formulae (i), (iii), or (v), and most preferably represented by the formula (i). Z³ in the general formulae (VI) for example, may be preferably represented by any one of groups represented by the abovementioned formulae (i) to (v), more preferably represented by any of formulae (i), (iii), or (v), and most preferably represented by the formula (v). Thiophene rings, furan rings and pyrrole rings, and in particular thiophene rings, demonstrate a distinctive electric quality, with various electric properties being exhibited thereby.

In formulae (i), (ii), and (iii), and in general formulae (I), (II), (III), (IV), and (VI), R¹ to R⁴ and R⁷ to R¹² each independently represent a hydrogen atom, a halogen atom, or a monovalent group, with the proviso that a ring may also be formed between R⁷ and R⁸, and between R¹¹ and R¹².

As the monovalent group that is R¹ to R⁴ and R⁷ to R¹², a straight-chain or branched low molecular weight chain, or a monovalent cyclic group (this monovalent cyclic group may be a single ring or fused ring, a carbon ring or heterocyclic ring, saturated or unsaturated, with or without a substituent) is preferable. The monovalent group may also be an electron-donating group or an electron-withdrawing group.

Moreover, as the monovalent group that is R¹ to R⁴ and R⁷ to R¹², a straight-chain or branched low molecular weight chain (one with a carbon number of 1 to 20), a monovalent cyclic group where the number of ring constituting atoms is 3 to 60 (this cyclic group may be a single ring or fused ring, a carbon ring or heterocyclic ring, saturated or unsaturated, with or without a substituent), a saturated or unsaturated hydrocarbon group, a hydroxy group, an alkoxy group, an alkanoyloxy group, an amino group, an oxyamino group, an alkylamino group, a dialkylamino group, an alkanoylamino group, a cyano group, a nitro group, a sulfo group, an alkyl group substituted by at least one halogen group, an alkoxysulfonyl group (however, the alkyl group thereof may be substituted by at least one halogen group), an alkylsulfonyl group (however, the alkyl group thereof may be substituted by at least one halogen group), a sulfamoyl group, an alkylsulfamoyl group, a carboxyl group, a carbamoyl group, an alkylcarbamoyl group, an alkanoyl group, or an alkoxycarbonyl group is more preferable.

Moreover, as the halogen atom of the present specification, a fluorine atom, a chlorine atom, a bromine atom, and an iodine atom may be exemplified.

Furthermore, the alkyl group is not particularly limited in any manner, and thus a methyl group, an ethyl group, an n-propyl group, an isopropyl group, an n-butyl group, an isobutyl group, a sec-butyl group and a tert-butyl group may be exemplified, with the same also applying to groups containing an alkyl group within a structure thereof (for example, an alkoxy group, a alkylamino group, and an alkoxycarbonyl group). As the alkyl group, an alkyl group with 1 to 12 carbon atoms is more preferable, and alkyl group with 1 to 10 carbon atoms is more preferable.

The unsaturated hydrocarbon is not particularly limited in any manner, and thus a vinyl group, a 1-propenyl group, an allyl group, a propargyl group, an isopropenyl group, a 1-butenyl group, and a 2-butenyl group may be exemplified. As the preferable unsaturated hydrocarbon, a vinyl group is exemplified.

Although the alkanoyl group is not particularly limited any manner, a formyl group, an acetyl group, a propionyl group, an isobutyryl group, a valeryl group, and an isovaleryl group may be exemplified, with the same also applying to groups containing an alkanoyl group within a structure thereof (for example, an alkanoyloxy group, and an alkanoylamino group). Moreover, an alkanoyl group with one carbon atom refers to a formyl group, and the same also applies to groups containing an alkanoyl group within a structure thereof. As the preferable alkanoyl group, a formyl group, and an acetyl group may be exemplified.

R¹ and R² in the general formulae (I), (II), (III), and (IV) are most preferably a halogen atom, and most preferably a fluorine atom, to thereby allow for suitable use in the thin film material of an organic thin film device as an organic n-type semiconductor. Specifically, the fluorine-containing polycyclic aromatic compound and the fluorine-containing polymer can be expected to contribute to the lowering of the cost of production and the improving of performance as an organic semiconductor, not only from the perspective of lowering the LUMO level via the introduction of fluorine atoms, but also from the perspective of increasing the solubility with respect to organic solvents and maintaining π-conjugated planarity and the like. The organic thin film device of the present invention is capable of achieving higher performance by containing the fluorine-containing polycyclic aromatic compound or the fluorine-containing polymer.

As R³ and R⁴ in the general formulae (I), (II), (III), and (IV), a hydrogen atom, a fluorine atom, an alkyl group, or an alkoxy group are preferable, with a hydrogen atom or a fluorine atom each independently being even more preferable.

As R⁵ and R⁶ in the general formulae (I), (II), (III), and (IV), a hydrogen atom, an alkyl group, an alkoxy group, an acyl group, a substituted or unsubstituted aryl group, a substituted or unsubstituted aryloxy group, a substituted or unsubstituted arylalkyl group, a substituted or unsubstituted arylalkoxy group, and a substituted or unsubstituted monovalent heterocyclic group are each independently preferable. As the substituent, a halogen atom, an alkyl group, a fluoroalkyl group, an alkoxy group, a fluoroalkoxy group, an acetyl group, a fluoroacetyl group, and an electron-donating group or an electron-withdrawing group may be exemplified, with a halogen atom, a fluoroalkyl group, a fluoroalkoxy group, a fluoroacetyl group, and an electron-withdrawing group being preferable.

As to R⁵ and R⁶ in the abovementioned general formulae (I) and (II), it is necessary that at least one of R⁵ and R⁶ is a group, in which one or more hydrogen atom of the group including the substituent is substituted with a fluorine atom. By including such a group, the LUMO level is lowered and the solubility towards organic solvents is increased. Form the perspective of increasing the electron transport property thereof, it is preferable that at least one of R⁵ and R⁶ is a fluoroalkyl group, a fluoroalkoxy group, a fluoroaryl group, an aryl group substituted by a fluoroalkyl group, an aryl group substituted by a fluoroalkoxy group, a heterocyclic group substituted by a fluoroalkyl group, or a monovalent heterocyclic group substituted by a fluoroalkoxy group, with it being especially preferable that both R⁵ and R⁶ are a fluoroalkyl group, a fluoroalkoxy group, a fluoroaryl group, an aryl group substituted by a fluoroalkyl group, an aryl group substituted by a fluoroalkoxy group, a monovalent heterocyclic group substituted by a fluoroalkyl group, or a monovalent heterocyclic group substituted by a fluoroalkoxy group.

The fluorine-containing polymer may include the repeating unit represented by the general formula (III) or (IV), or it may include at least two of the repeating units represented by the general formula (III) or (IV). Moreover, in addition to the repeating unit represented by the general formula (III) or (IV), it may include the repeating unit represented by the general formula (V) or (VI), or it may include at least two of the repeating units represented by the general formula (V) or (VI).

It is preferable that the fluorine-containing polymer of the present invention includes a structure in which the repeating unit represented by the abovementioned general formula (III) or (IV) and the repeating unit represented by the abovementioned general formula (V) or (VI) are adjacent. In cases where the repeating unit represented by the general formula (III) or (IV) and the repeating unit represented by the general formula (V) or (VI) are adjacent, the dihedral angle of both adjacent aromatic rings or heterocyclic rings can be reduced, the intramolecular planarity easily improved, the intramolecular π-conjugation widened, and the LUMO level lowered, to thereby improve the electron transport property. In such a case the dihedral angle is defined as an angle between no less than 0 degrees to no greater than 90 degrees and which is one from the angles formed between the plane containing the aromatic ring and the heterocyclic ring represented by the general formula (III) or (IV), and the plane containing the aromatic ring or the heterocyclic ring linked adjacent thereto. In cases where the repeating unit represented by the abovementioned general formula (III) or (IV) and the repeating unit represented by the abovementioned general formula (V) or (VI) are adjacent, the dihedral angle is normally 0 degrees to 45 degrees, typically 0 degrees to 40 degrees, and even more typically 0 degrees to 30 degrees.

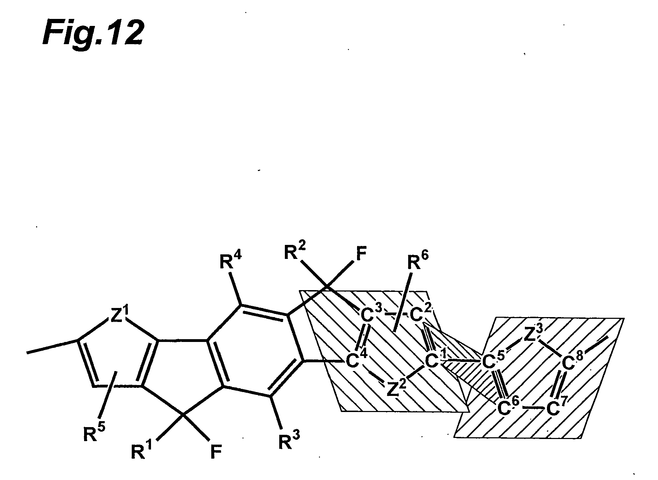

FIG 12 is a diagram showing a dihedral angle formed by a ring of a repeating unit represented by the general formula (IV) and a ring of a repeating unit represented by the general formula (VI). The dihedral angle refers to an angle formed between the plane formed by C²-C¹-C⁵ and the plane formed by C¹-C⁵-C⁶ in FIG 12.

Moreover, it is preferable that the fluorine-containing polymer of the present invention is one represented by the below-mentioned general formulae (VIII), (IX), and (X) from the perspective of increasing the electron transport property.

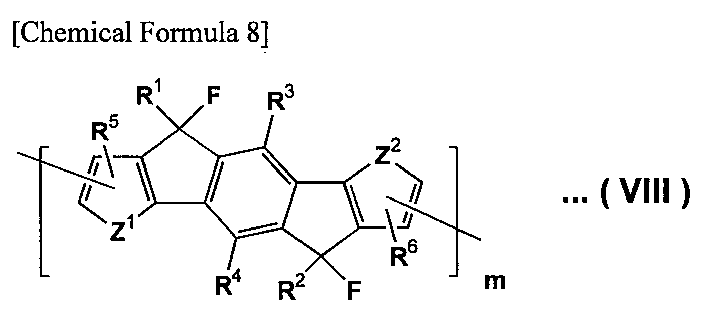

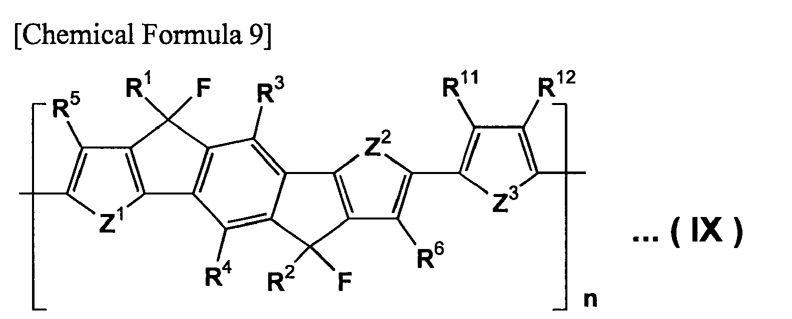

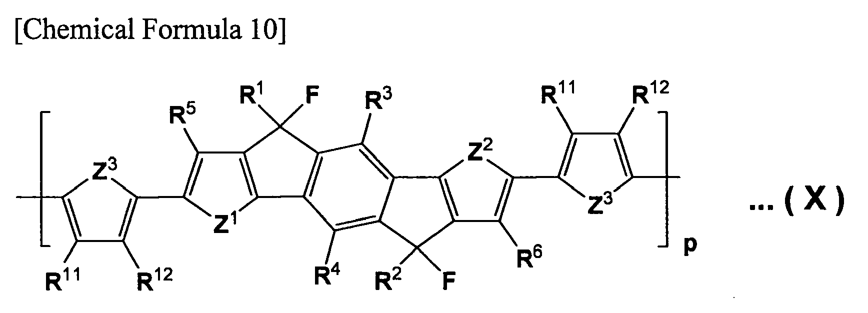

In general formulae (VIII) to (X), Z¹, Z², Z³, R¹, R², R³, R⁴, R⁵, R⁶, R¹¹, and R¹² are the same as above in meaning. Moreover, in cases where a plurality of these are present, Z¹, Z², Z³, R¹, R², R³, R⁴, R⁵, R⁶, R¹¹, and R¹² may each be the same or different. m represents an integer of 2 to 500, which is preferably an integer of 2 to 100, more preferably an integer of 3 to 20. n represents an integer of 1 to 500, which is preferably 1 to 100, and more preferably 2 to 200. p represents an integer of 1 to 500, which is preferably 1 to 100, and more preferably 1 to 10. Among these, it is especially preferable that Z¹, Z² and Z³ are all represented by the formula (i) and that R¹ and R² are fluorine atoms.

Furthermore, in cases where a polymerization active group is included as the terminal group of the fluorine-containing polymer, this group may also be used as precursor for a fluorine-containing polymer. In such cases, it is preferable that the fluorine-containing polymer has two intramolecular polymerization active groups. As the polymerization active group, a halogen atom, an alkyl sulfonate group, an aryl sulfonate group, an arylalkyl sulfonate group, an alkylstannyl group, an arylstannyl group, an arylalkylstannyl group, a boric acid ester residue group, a sulfoniummethyl group, a phosphoniummethyl group, a phosphonatemethyl group, a monohalogenated methyl group, a boric acid residue group, a formyl group, or a vinyl group may be exemplified, with a halogen atom, an alkylstannyl group, and a boric acid ester residue group being preferable. In such cases, the boric acid residue group represents a group in which a hydroxy group is substituted at boron, and the boric acid ester residue group represents a group in which an alkyloxy group is substituted at boron. As the boric acid ester residue group the below-mentioned formulae (α) to (δ) may be exemplified.

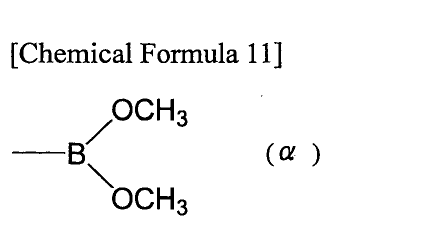

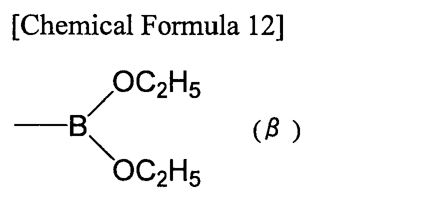

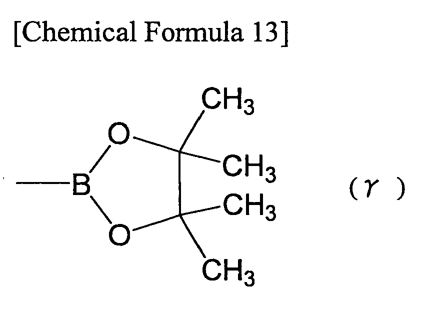

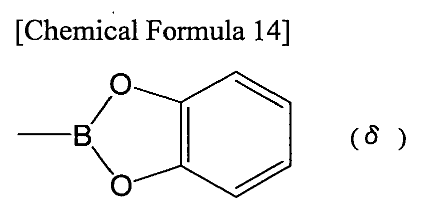

Moreover, in cases where the fluorine-containing polymer of the present invention is used as an organic thin film, and the polymerization active group at the terminal group remains as is, such a group may be protected by a stable group so as to avoid a potential reduction in durability or properties of the device formed therefrom.

As the terminal group, a hydrogen atom, a fluorine atom, an alkyl group, an alkoxy group, an acyl group, an aminoketo group, an aryl group, a monovalent heterocyclic group (some or all of the hydrogen atoms linked to these groups may be substituted with a fluorine atom), and an electron-donating group or an electron-withdrawing group may be exemplified; among which a fluoroalkyl group, a fluoroalkoxy group, a fluoroaryl group or an electron-withdrawing group is preferable, and a group in which all the hydrogen atoms are substituted by a fluorine atom, for example, a perfluoroalkyl group, a perfluoroalkoxy group, and a perfluorophenyl group is more preferable, from a standpoint of increasing the electron transport property. In addition, including conjugated bonds that are continuous with the conjugated structure of the main chain is preferable, and as such, a structure bonding with an aryl group or a monovalent heterocyclic group via a carbon-carbon bond may be exemplified.

Particularly preferable examples of the fluorine-containing polymer of the present invention may be represented by the below-mentioned general formulae (1) to (5).

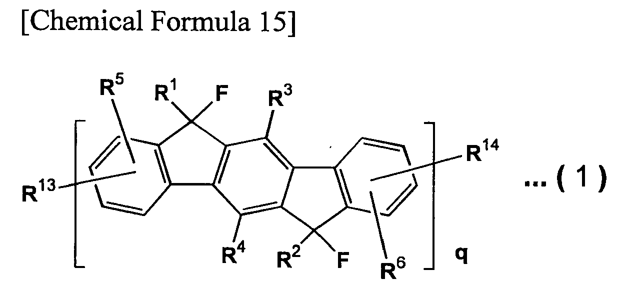

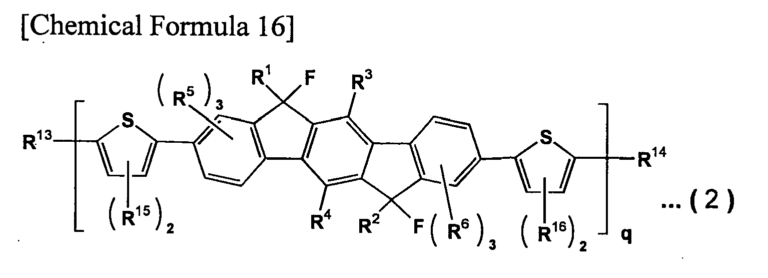

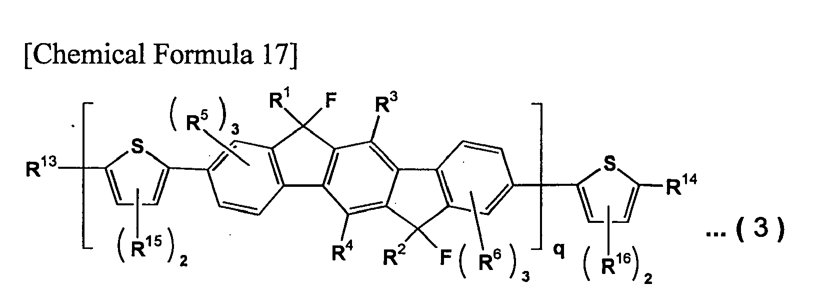

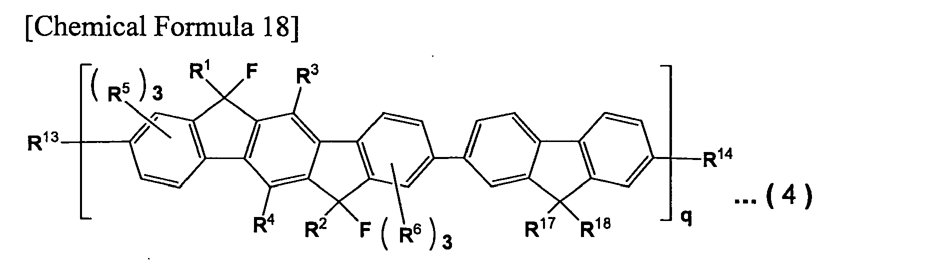

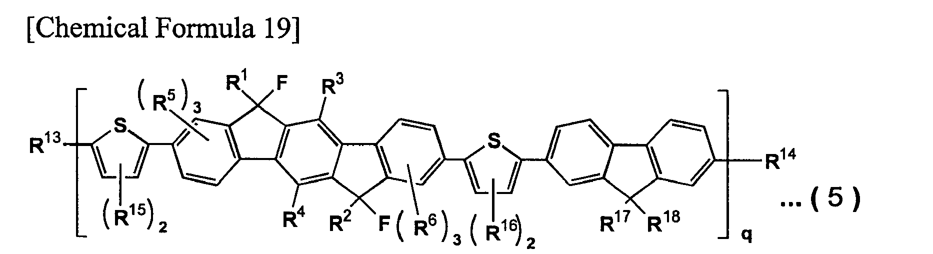

In the general formulae (1) to (5), R¹³ and R¹⁴ represent terminal groups, where these may be the same as or different from each other, where the above-illustrated terminal groups are provided as examples, where a fluoroalkyl group is preferable, and where a perfluoroalkyl group is more preferable. R¹⁵, R¹⁶, R¹⁷, and R¹⁸ each independently represent a hydrogen atom or an arbitrary substituent, with an alkyl group, an alkoxy group, or an aryl group being preferable, and an alkyl group being more preferable. In cases where there are a plurality of R¹⁵, R¹⁶, R¹⁷, and R¹⁸ present in the fluorine-containing polymer, these may be either the same or different. Moreover, in order to facilitate the production thereof, it is preferable that the plurality of R¹⁵, R¹⁶, R¹⁷, and R¹⁸ present are the same. Furthermore, R¹, R², R³, R⁴, R⁵, and R⁶ are the same as above in meaning. q may be arbitrarily selected in accordance with the formation method of the organic thin film employing the fluorine-containing polymer. In cases where the organic thin film can be formed using a vapor growth method such as a vacuum deposition method when the fluorine-containing polymer is sublimable, q is preferably 1 to 10, more preferably 2 to 10, and even more preferably 2 to 5. On the other hand, in cases where the organic thin film is formed by employing a method of coating a solution with the fluorine-containing polymer dissolved in an organic solvent, q is preferably is preferably 3 to 500, more preferably 6 to 300, and even more preferably 20 to 200. From the standpoint of the homogeneity of the film when formed by coating, the number-average molecular weight in terms of the polystyrene of the fluorine-containing polymer is preferably 1×10³ to 1×10⁸, and more preferably 1×10⁴ to 1×10⁶.

As a specific example of the fluorine-containing polycyclic aromatic compound and the fluorine-containing polymer of the present invention, the following are exemplified.

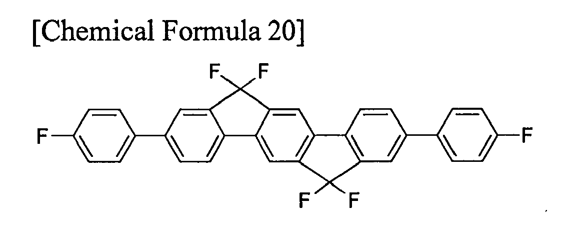

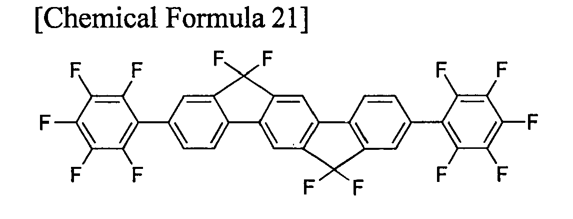

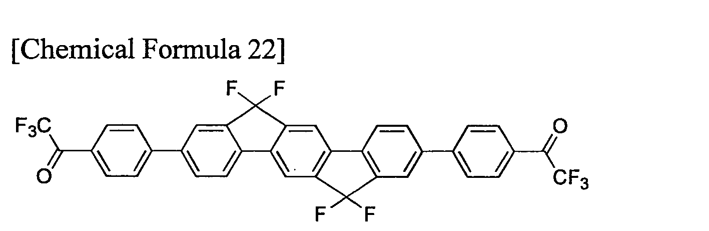

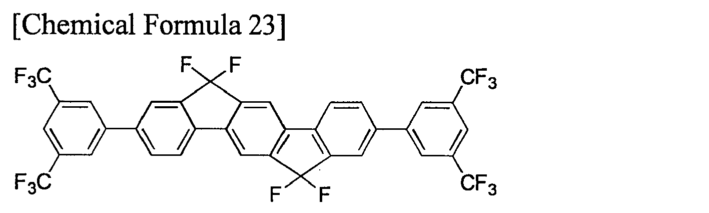

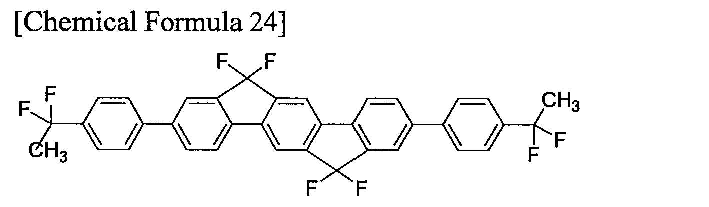

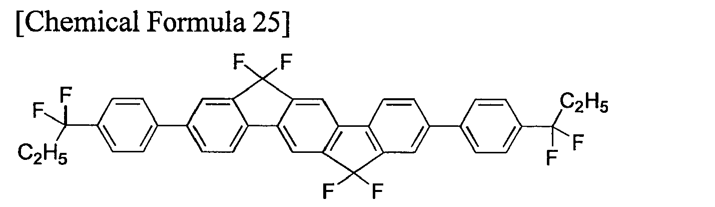

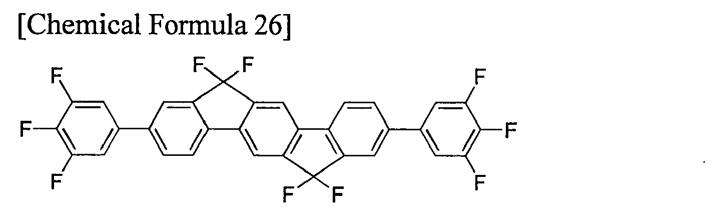

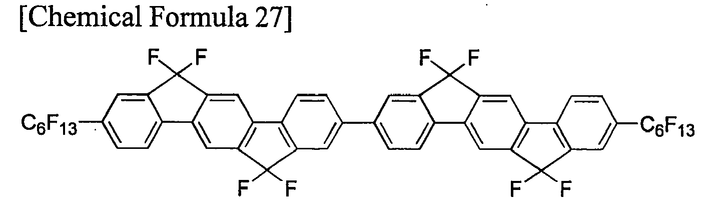

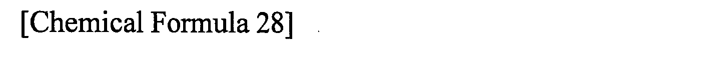

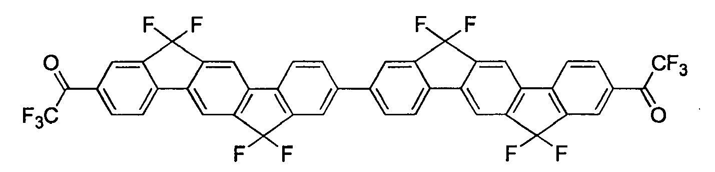

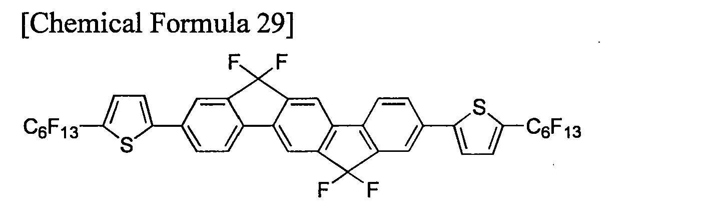

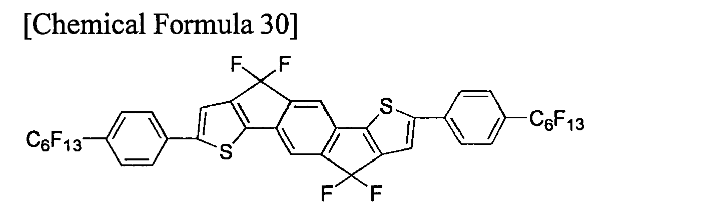

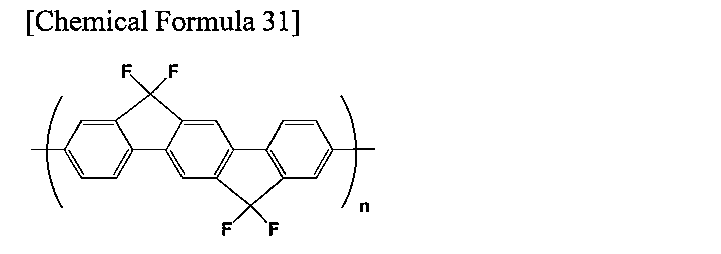

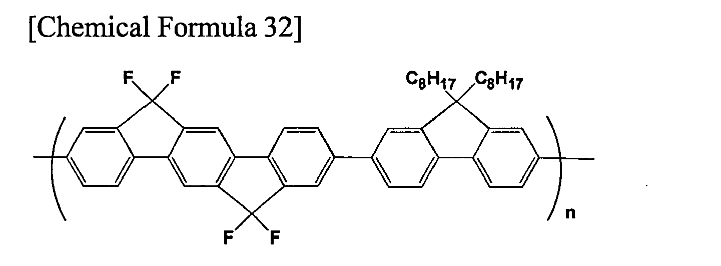

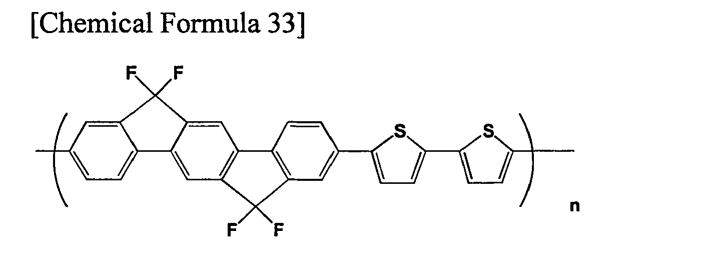

A production method of the fluorine-containing polycyclic aromatic compound or the fluorine-containing polymer of the present invention is not particularly limited in any manner. However, regardless of the fact that any method may be employed as the production method thereof, production by the production method described below is preferable.

First, the production method the fluorine-containing polycyclic aromatic compound of the present will be described. The fluorine-containing polycyclic aromatic compound represented by the abovementioned general formula (I) may be produced by a production method including a fluorinating step which uses the compound represented by the below-mentioned general formula (VII) as a precursor and reacts this precursor with a fluorinating agent. Specifically, the compound represented by the abovementioned general formula (I) may be produced by a production method including a step which uses the compound represented by the below-mentioned general formula (VII) as a precursor and reacts the abovementioned precursor with a fluorinating agent, for example, in a manner similar to the method of alcohol fluorination described in Organic Reactions vol. 35, p. 315 (1988) Chap. 3, "Fluorination with DAST". As an example this reaction step, the conversion step of compound (6) into compound (7) indicated in the below-mentioned reaction formula (a) may be exemplified.

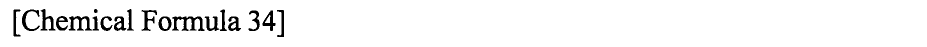

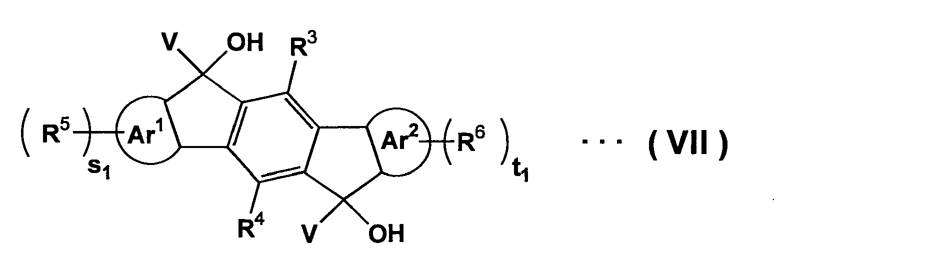

Moreover, in the abovementioned general formula (VII), Ar¹, Ar², R³, R⁴, R⁵, R⁶, s₁, and t₁ are the same as above in meaning, and V represents a hydrogen atom, a halogen atom, or a monovalent group.

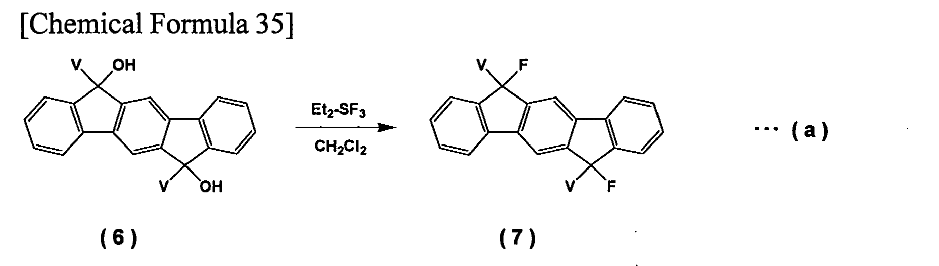

Furthermore, among the fluorine-containing polycyclic aromatic compounds of the present invention represented by the abovementioned general formula (I), a compound in which R¹ and R² is a fluorine atom is preferably produced by a method including a step that uses a compound represented by the below-mentioned general formula (b) as a precursor and reacts this precursor with a fluoride ion source in the presence of a halonium ion generator, a production method including a step of halogenating a hydrogen atom bonded to Ar¹ and Ar², and method including a step of reacting a halogen atom with the below-mentioned general formula (c). As an example of this reaction step, the conversion steps of compound (8), into compound (9), into compound (11), and into compound (12), or the conversion steps from compound (8), into compound (10), and into compound (12), as indicated in the below-mentioned reaction formula (d), may be exemplified.

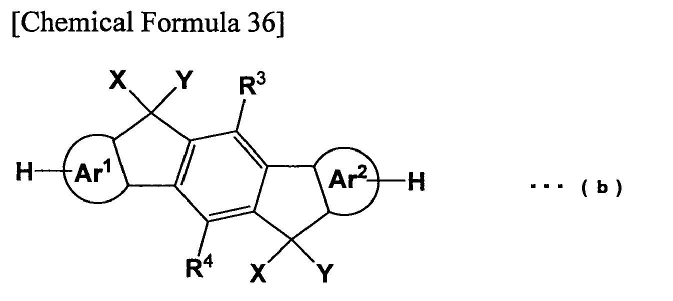

Moreover, in the general formula (b), Ar¹ and Ar² each independently represent an aromatic hydrocarbon group having at least six carbon atoms or a heterocyclic group having at least four carbon atoms; and R³ and R⁴ represent a hydrogen atom, a halogen atom, or a monovalent group. X and Y each independently represent an alkylthio group (where X and Y may form a alkylenedithio linked by the alkyl portions, or X and Y may unite to form a thiocarbonyl group together with the carbon atoms to which they are linked).

[Chemical Formula 37] R^{x}-W (c)

Furthermore, in the abovementioned (c), R^{x} represents R⁵ or R⁶ as represented by the general formula (1), and W represents a polymerization active group.

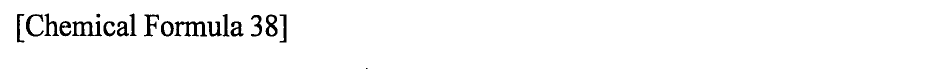

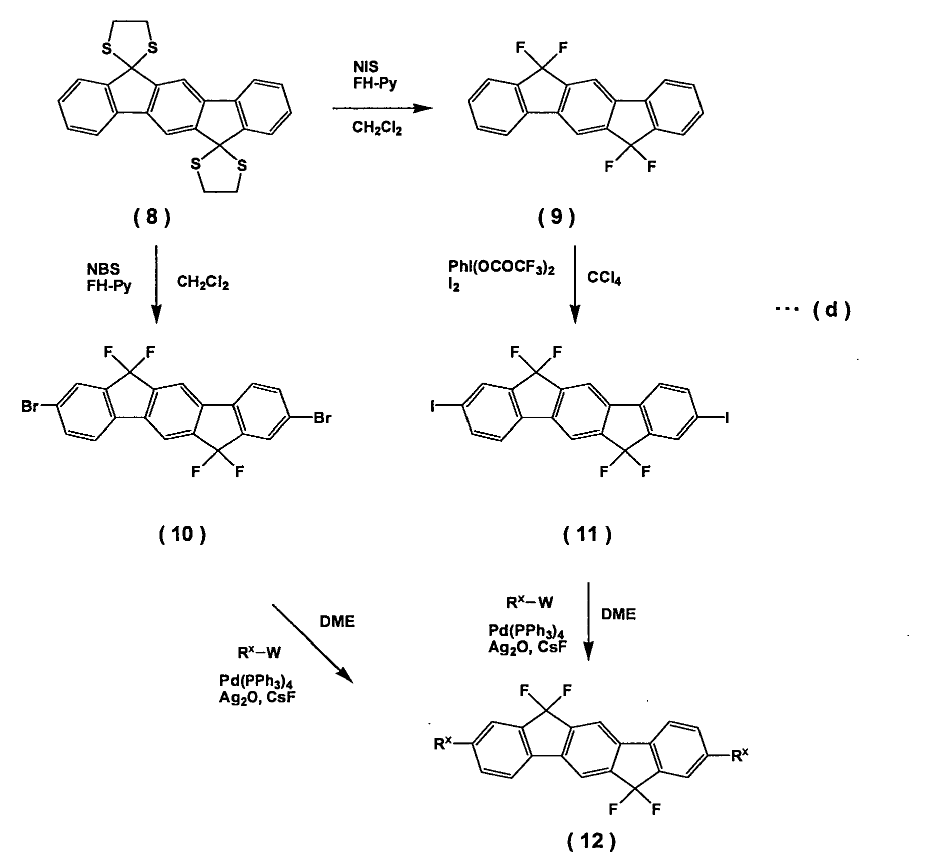

The reaction conditions in the abovementioned reaction step employing the compound represented by the abovementioned general formula (b) as the precursor are not particularly limited in any manner. For example, the conditions may be arbitrarily selected with reference to conditions of known conversion reactions (refer to Japanese Patent Application Laid-Open Publication No. H06-135869) for conversion from an alkylsulfanyl group and the like (alkylthio group) to a fluoro group. Hereinafter, the details thereof will be described.

As the abovementioned halonium ion generator of the abovementioned reaction step, any known halonium ion generator may be arbitrarily selected. For example, 1,3-dibromo-5,5-dimethylhydantoin (DBH); N-bromosuccinic acid imide (NBS); N-bromoacetamide (NBA); 2,4,4,6-tetrabromo-2,5,cyclohexadienone; N-iodosuccinic acid imide (NIS) may be exemplified. Although the amount of the abovementioned halonium ion generator used is not particularly limited in any manner, it is in the range of 3 equivalents to a highly excess amount for example, and among these preferably 3 equivalents to 5 equivalents from the viewpoint of reaction efficiency and cost.

As the abovementioned fluoride ion source, hydrogen fluoride, a complex of a hydrogen fluoride and an amine, a complex of a hydrogen fluoride and pyridine, a quaternary ammonium dihydrogen trifluoride, or a quaternary phosphonium dihydrogen trifluoride are preferable, either alone or in combinations of two or more. As a suitable fluoride ion source, (hydrogen fluoride)₉/pyridine complex may be exemplified. Furthermore, although the amount of the abovementioned fluoride ion source used is not particularly limited in any manner, it is in the range of 3 equivalents to a highly excess amount for example, and among these preferably 3 equivalents to 5 equivalents from the viewpoint of reaction efficiency and cost.

Moreover, a nitrogen-containing cyclic compound such as pyridine; and an alkylamine such as triethylamine, and diisopropylethylamine may be exemplified as the amine. Furthermore, the quaternary ammonium dihydrogen trifluoride and the quaternary phosphonium dihydrogen trifluoride are not particularly limited in any manner, and as such any known compound may be appropriately employed. A compound represented by the below-mentioned general formula (XI) may be exemplified as the quaternary ammonium dihydrogen trifluoride, and a compound represented by the below-mentioned general formula (XII) may be exemplified as the quaternary phosphonium dihydrogen trifluoride.

R¹⁹R²⁰R²¹R²²N⁺H₂F₃⁻ (XI)

R²³R²⁴R²⁵R²⁶P⁺H₂F₃⁻ (XII)

In the abovementioned general formula (XI) and (XII), R¹⁹, R²⁰, R²¹, R²², R²³, R²⁴, R²⁵, and R²⁶ each independently represent a hydrocarbon group such as an alkyl group, an aryl group, or a benzyl group. As the quaternary ammonium dihydrogen trifluoride represented by the general formula (XI), tetramethyammonium dihydrogen trifluoride, tetraethylammonium dihydrogen trifluoride, tetrabutylammonium dihydrogen trifluoride (TBAH2F3), benzyltrimethylammonium dihydrogen trifluoride, benzyltriethylammonium dihydrogen trifluoride, and cetyltrimethylammonium dihydrogen trifluoride may be exemplified. These may be readily synthesized, for example, from 50% hydrofluoric acid, potassium fluoride, and quaternary ammonium fluoride (for example, refer to Bull. Soc. Chim. Fr., 910 (1986)). In addition, as the quaternary phosphonium dihydrogen trifluoride represented by the abovementioned general formula (XII), tetramethylphosphonium dihydrogen trifluoride, tetraethylphosphonium dihydrogen trifluoride, tetrabutylphosphonium dihydrogen trifluoride, benzyltrimethylphosphonium dihydrogen trifluoride, benzyltriethylphosphonium dihydrogen trifluoride, and cetyltrimethylphosphonium dihydrogen trifluoride may be exemplified.

Furthermore, a solvent also may be appropriately employed where necessary in the abovementioned reaction step. Although the abovementioned solvent is not particularly limited in any manner, it is preferably be one that does not interfere much with the intended reaction. For example, an aliphatic hydrocarbon such as hexane; an aromatic hydrocarbon such as benzene, and toluene; a nitrile such as acetonitrile; an ether such diethyl ether, tetrahydrofuran, and 1,2,-dimethoxyethane; and halogenated solvents such as dichloromethane, 1,2-dichloroethane, and carbon tetrachloride may be exemplified. These may be employed alone or in combinations of two or more. As the preferred solvent, dichloromethane may be exemplified.

The reaction temperature and reaction may be appropriately selected in view of the type of precursor, without any particular limitations being applied thereto. In addition, it is preferable, for example, that the abovementioned reaction temperature is a range of -100°C to 100°C.

The abovementioned production method of the present invention allows for the simple and high yield production of a cyclic compound cross-linked with a fluoroalkyl group, and particularly a compound including a difluoroalkyl cross-linked terphenyl structure, which would have been difficult to obtain by conventional art. Moreover, fluorination in the fluorination reaction of the production step of the present invention may be performed by employing an inexpensive and easily handled fluorinating reagent.

In cases where the fluorine-containing polycyclic aromatic compound of the present invention is employed as the material of an organic thin film device, it is preferable that the compound produced is purification treated by a method such as distillation, sublimation purification, and recrystallization, since the device characteristics are affected by the purity thereof.

The reaction conditions, reaction reagent, and the like, of the abovementioned production method, other than that illustrated above, may also be arbitrarily selected. In addition, as mentioned above, although the fluorine-containing polycyclic aromatic compound of the present invention represented by the abovementioned general formula (I) is preferably produced by the abovementioned production method, it may be produced by any method without any particular limitation applying thereto.

Next, the production method of the fluorine-containing polymer of the present invention will be described. The fluorine-containing polymer may be produced, for example, by reacting the compounds represented by the below-mentioned formulae (XII) to (XVI) as the raw materials.

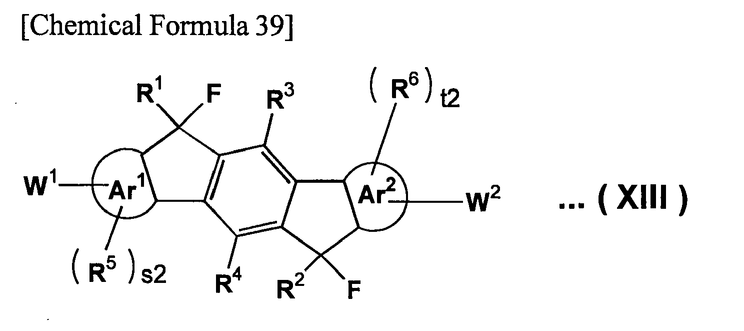

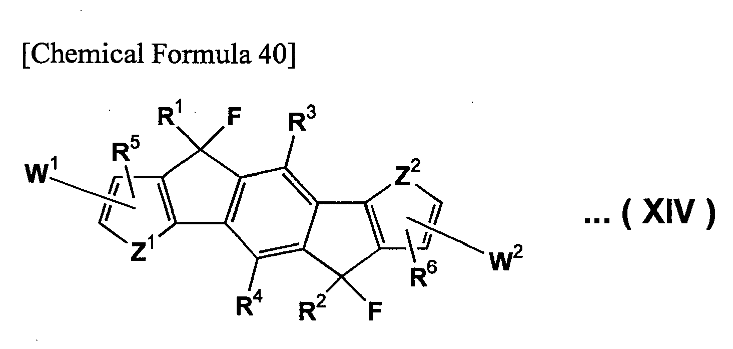

[Chemical Formula 41] W¹-Ar³-W² (XV)

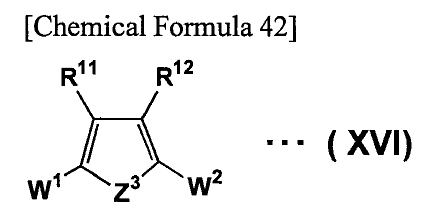

In the abovementioned general formulae (XIII) to (XVI), Ar¹, Ar², Ar³, Z¹, Z², Z³, R¹, R², R³, R⁴, R⁵, R⁶, R¹¹, R¹², s², and t² are the same as above in meaning. W¹ and W² each independently represent a halogen atom, an alkyl sulfonate group, an aryl sulfonate group, an arylalkyl sulfonate group, an alkylstannyl group, an arylstannyl group, an arylalkylstannyl group, a boric acid ester residue group, a sulfoniummethyl group, a phosphoniummethyl group, a phosphonatemethyl group, a monohalogenated methyl group, a boric acid residue group, a formyl group, or a vinyl group.

From the standpoint of the facilitation of the reaction and synthesis of the compound represented by the general formulae (XIII) to (XVI), it is preferable that W¹ and W² are each independently a halogen atom, an alkyl sulfonate group, an aryl sulfonate group, an arylalkyl sulfonate group, an alkylstannyl group, a boric acid ester residue group, or a boric acid residue group.

Moreover, as the reaction method employed in the production method of the fluorine-containing polymer of the present invention, a method employing the Witting reaction, a method employing the Heck reaction, a method employing the Horner-Wadsworth-Emmons reactions, a method employing the Knoevenagel reaction, a method employing the Suzuki coupling reaction, a method employing the Grignard reaction, a method employing the Stille reaction, a method employing an Ni(0) catalyst, a method employing oxidizing agent such as FeCl₃, a method employing electrochemical oxidative reaction, or a method whereby the degradation of an intermediate compound having the appropriate elimination group is performed may be exemplified.

Among these, a method employing the Witting reaction, a method employing the Heck reaction, a method employing the Horner-Wadsworth-Emmons reactions, a method employing the Knoevenagel reaction, a method employing the Suzuki coupling reaction, a method employing the Grignard reaction, a method employing the Stille reaction, and a method employing the Ni(0) catalyst are preferable for facilitating structural control. Furthermore, a method employing the Suzuki coupling reaction, a method employing the Grignard reaction, a method employing the Stille reaction, and a method employing the Ni(0) catalyst are preferable for easy availability as a raw material and simplification of the reaction process.

A monomer (the compound represented by the abovementioned general formulae (XIII) to (XVI)), may be dissolved in an organic solvent where necessary, and reacted between the melting point and boiling point, for example, using an alkali or appropriate catalyst.

Although the organic solvent may differ depending on the reaction or compound employed, it is generally preferable that the solvent employed is one that sufficiently accomplishes a deoxygenation treatment and promotes the reaction under an inert atmosphere, in order to control side reactions. Furthermore, it is similarly preferable that a dehydration treatment is performed (however, this does not apply to cases where there is a two-phase reaction with water such the Suzuki coupling reaction).

A suitable alkali or appropriate catalyst is added for the reaction. These may be selected where necessary for the reaction employed. The alkali or catalyst is preferably one that sufficiently dissolves in the solvent employed in the reaction.

In cases where the fluorine-containing polymer of the present invention is employed as the material for an organic thin film device, it is preferable that the monomer of the abovementioned reaction is polymerized after being purified by a method such as distillation, sublimation purification, and recrystallization, since the device characteristic features are affected by the purity thereof. Then, after synthesis of the fluorine-containing polymer, performing a purification treatment such as repreciptating purification, or separation by chromatography is preferable.

As the solvent employed in the reaction, a saturated hydrocarbon such as pentane, hexane, heptane, octane, and cyclohexane; an unsaturated hydrocarbon such as benzene, toluene, ethylbenzene, and xylene; a halogenated saturated hydrocarbon such as carbon tetrachloride, chloroform, dichloromethane, chlorobutane, bromobutane, chloropentane, bromopentane, chlorohexane, bromohexane, chlorocyclohexane, and bromocyclohexane; a halogenated unsaturated hydrocarbon such as chlorobenzene, dichlorobenzene, and trichlorobenzene; an alcohol such as methanol, ethanol, propanol, isopropanol, butanol, and t-butyl alcohol; a carboxylic acid such as formic acid, acetic acid, and propionic acid; an ether such as dimethyl ether, diethyl ether, methyl-t-butyl ether, tetrahydrofuran, tetrahydropyran, and dioxane; and an inorganic acid such as hydrochloric acid, hydrobromic acid, hydrofluoric acid, sulfuric acid, and nitric acid may be exemplified. The abovementioned solvent may be employed either alone or in combinations of two or more.

The reaction may be followed by a conventional post-treatment in which extraction is performed with an organic solvent after quenching with water and the solvent is distilled, for example. The purification and post-isolation of the product may be performed by a method such as fractionation by chromatography or recrystallization.

Next, the organic thin film of the present invention will be described. The organic thin film of the present invention is one that includes the fluorine-containing polycyclic aromatic compound and/or the fluorine-containing polymer of the present invention (hereinafter, both of these will be referred to as "fluorine-containing compound of the present invention").

The film thickness of the organic thin film is normally approximately 1 nm to 100 µm, preferably 2 nm to 1000 µm, more preferably 5 nm to 500 µm, and most preferably 20 nm to 200 µm.

The organic thin film may be one including one type of the fluorine-containing compound of the present invention alone or it may include at least two types of the fluorine-containing compound of the present invention. Furthermore, in addition to the fluorine-containing compound, a low molecular weight compound or a high molecular weight compound with an electron transport property or hole transport property may also be compounded and employed, in order to improve the hole transport property or the electron transport property of the organic thin film.

As the hole transport property material, a known material may be employed. Thus, a pyrazoline derivate, an arylamine derivative, a stilbene derivative, a triphenyldiamine derivative, oligothiophene or a derivative thereof, polyvinylcarbazole or a derivative thereof, polysilane or a derivative thereof, a polysiloxane derivative with an aromatic amine on a side chain or main chain, polyaniline or a derivative thereof, polythiophene or a derivative thereof, polypyrrole or a derivative thereof, polyarylenevinylene or a derivative thereof, and polythienylenevinylene or a derivative thereof may be exemplified. As the electron transport property material, a known material may be employed. Thus, a oxadiazole derivative, anthraquinodimethane or a derivative thereof, benzoquinone or a derivative thereof, naphthoquinone or a derivative thereof, anthraquinone or a derivative thereof, tetracyanoanthraquinodimethane or a derivative thereof, a fluorenone derivative, diphenyldicyanoethylene or a derivative thereof, a diphenoquinone derivative, or a metal complex of 8-hydroxyquinoline or a derivative thereof, polyquinoline or a derivative thereof, polyqiunoxaline or a derivative thereof, polyfluorene or a derivative thereof, and a fullerene such as C₆₀ or a derivative thereof may be exemplified.

In addition, the organic thin film of the present invention may also include a charge generation material, in order to generate an electric charge via light absorbed within the organic thin film. As the charge generation material a known material may be employed. Thus, an azo compound or a derivative thereof, a diazo compound or a derivative thereof, a metal-free phthalocyanine compound or a derivative thereof, a metallophthalocyanine compound or a derivative thereof, a perylene compound or a derivative thereof, a polycyclic quinone compound or a derivative thereof, a squarlyium compound or a derivative thereof, a azulenium compound or a derivative thereof, a thiapyrylium compound or a derivative thereof, and a fullerene such as C₆₀ or a derivative thereof may be exemplified.

Moreover, the organic thin film of the present invention may also include materials required for exerting various functions. Thus, a sensitizing agent for sensitizing a function for generating an electric charge via absorbed light, a stabilizing agent for increasing stability, and a UV absorber for absorbing UV light may be exemplified.

Furthermore, the organic thin film of the present invention may include a high molecular weight compound other than the fluorine-containing compound of the present invention as a high molecular weight binder in order to increase the mechanical properties thereof. The high molecular weight binder that is preferably employed is one that provides minimal interference with the electron transport property or hole transport property, and also preferably one that demonstrates weak absorption of visible light.

As high molecular weight compound, poly(N-vinylcarbazole), polyaniline or a derivative thereof, polythiophene or a derivative thereof, poly(p-phenylenevinylene) or a derivative thereof, poly(2,5-thienylenevinylene) or a derivative thereof, polycarbonate, polyacrylate, polymethyl acrylate, polymethyl methacrylate, polystyrene, polyvinyl chloride, and polysioxane may be exemplified.

The production method of the organic thin film of present invention is not particularly limited. For example, a method provided whereby film formation is from a solution including the fluorine-containing compound of the present invention, and where necessary, an electron transport property material or hole transport property material and high molecular weight binder to be combined therewith. Moreover, the thin film may also be formed by vacuum deposition method in cases where the fluorine-containing compound of the present invention is sublimable.

The solvent employed in film formation from a solution is not particularly limited in any manner so long as the fluorine-containing compound, and the electron transport property material or hole transport property material and high molecular weight binder combined therewith are dissolved.

As the solvent employed when performing film formation from a solution of the organic thin film of the present invention, an unsaturated hydrocarbon solvent such as toluene, xylene, mesitylene, tetralin, decalin, bicyclohexyl, n-butylbenzene, sec-butylbenzene, and tert-butylbenzene; a halogenated saturated hydrocarbon solvent such as carbon tetrachloride, chloroform, dichloromethane, dichloroethane, chlorobutane, bromobutane, chloropentane, bromopentane, chlorohexane, bromohexane, chlorocyclohexane, and bromocyclohexane; a halogenated unsaturated hydrocarbon solvent such as chlorobenzene, dichlorobenzene, and trichlorobenzene; and an ether solvent such as tetrahydrofuran and tetrahydropyran may be exemplified. Although varying depending on the molecular weight or structure of the fluorine-containing compound of the present invention, at least 0.1 wt % may normally be dissolved in these solvents.

A coating method such as a spin coating method, a casting method, a microgravure coating method, a gravure coating method, a bar coating method, a roll coating method, a wire bar coating method, a dip coating method, a spray coating method, a screen printing method, a flexographic printing method, an offset printing method, an inkjet printing method, a dispenser printing method, a nozzle coating method, and a capillary coating method may be employed in the film formation from a solution, with a spin coating method, a flexographic printing method, an inkjet printing method, a dispenser printing method, a nozzle coating method, and a capillary coating method being preferable.

A step of orienting the fluorine-containing compound of the present invention may also be included in the step of producing the organic thin film of the present invention. An organic thin film with a fluorine-containing compound oriented by this step has an electron mobility or hole mobility thereof improved, as a result of having the main chain molecules and side chain molecules are aligned in one direction.

As a method for orienting the fluorine-containing compound, a method known as an orientation method for crystals may be employed. Among such methods, a rubbing method, a photoorientation method, a shearing method (shear stress application method), or a pull-up coating method are simple, useful, and convenient as an orientation method, with a rubbing method and a shearing method being preferable.

Since the organic thin film of the present invention has an electron transport property or a hole transport property, the transport of electrons or holes introduced from an electrode, or of an electric charge generated via light absorption can be controlled to thereby allow for use of various organic thin film devices such as an organic thin film transistor, an organic solar cell, and a photosensor. In cases where the organic thin film of the present invention is employed in these organic thin film devices, it is preferable that it is oriented and employed via an orientation treatment, in order to further improve the electron transport property or hole transport property thereof.

Next, the applications of the organic thin film of the present invention to an organic thin film transistor will be described. The organic thin film transistor may have a structure that includes a source electrode and a drain electrode, an organic thin film layer (active layer) including the fluorine-containing compound of the present invention that forms a current channel therebetween, and a gate electrode controlling the amount of current flowing through the current channel, with a field effect type, static induction type, or the like being provided as an example thereof.

A field effect type organic thin film transistor is preferably provided with a source electrode and a drain electrode, an organic thin film layer (active layer) including the fluorine-containing compound of the present invention that forms a current channel therebetween, a gate electrode controlling the amount of current flowing through the current channel, and an insulating layer arranged between the active layer and gate layer. Specifically, it is preferable that the source electrode and the gate electrode are provided in contact with the organic thin film layer (active layer) including the fluorine-containing compound of the present invention, and that the gate electrode is provided by sandwiching the insulating layer also in contact with the organic thin film layer.

A static induction type organic thin film transistor includes a source electrode and a drain electrode, an organic thin film layer including the fluorine-containing compound of the present invention that forms a current channel therebetween, and a gate electrode controlling the amount of current flowing through the current channel, with the gate electrode being preferably provided in the organic thin film layer. Specifically, it is preferable that the source electrode, the drain electrode, and the gate electrode provided in the organic thin film layer, are provided in contact with the organic thin film layer including the fluorine-containing compound of the present invention. The structure of the gate electrode may be one with a current channel formed to flow from the source electrode to the drain electrode, and the amount of current flowing through the current channel being controllable via the voltage applied to the gate electrode, and comb-shaped electrode may be exemplified.

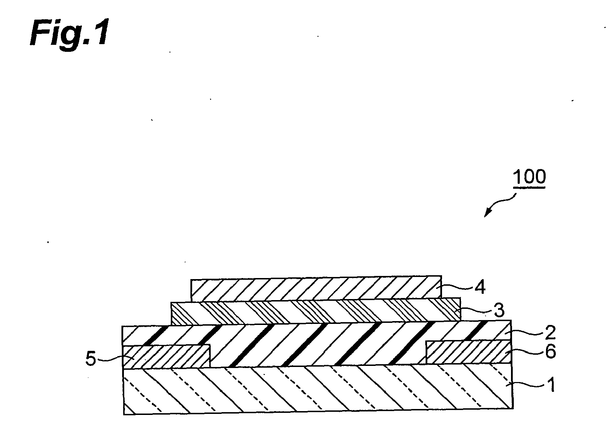

FIG. 1 is a schematic cross-sectional diagram of an organic thin film transistor (field effect type organic thin film transistor) according to a first embodiment of the present invention. The organic thin film transistor 100 shown in FIG 1 is provided with a substrate 1; a source electrode 5 and a drain electrode 6 formed at a predetermined distance on substrate 1; an active layer 2 formed on top of substrate 1, so as to cover the source electrode 5 and the drain electrode 6; an insulating layer 3 formed on the active layer 2; and a gate electrode 4 formed on the insulating layer 3, so as to cover a region of the insulating layer 3 between the source electrode 5 and the drain electrode 6.

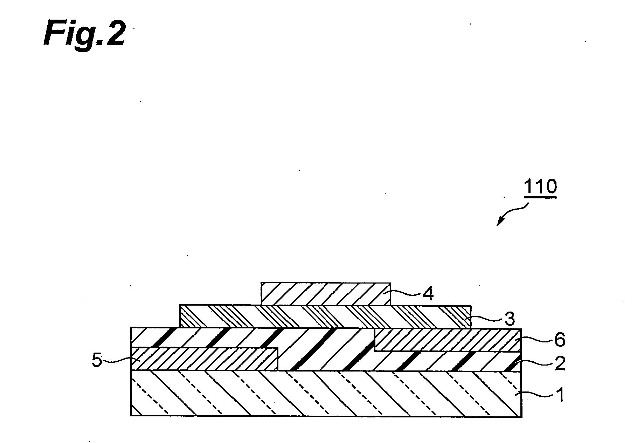

FIG 2 is a schematic cross-sectional diagram of an organic thin film transistor (field effect type organic thin film transistor) according to a second embodiment of the present invention. The organic thin film transistor 110 shown in FIG. 2 is provided with a substrate 1; a source electrode 5 formed on the substrate 1; an active layer 2 formed on the substrate 1, so as to cover the source electrode 5; a drain electrode 6 formed on the active layer 2, so as to be at a predetermined distance from the source electrode 5; an insulating layer 3 formed on the active layer 2 and the drain electrode 6; and a gate electrode 4 formed on the insulating layer 3, so as to cover a region of the insulating layer 3 between the source electrode 5 and the drain electrode 6.

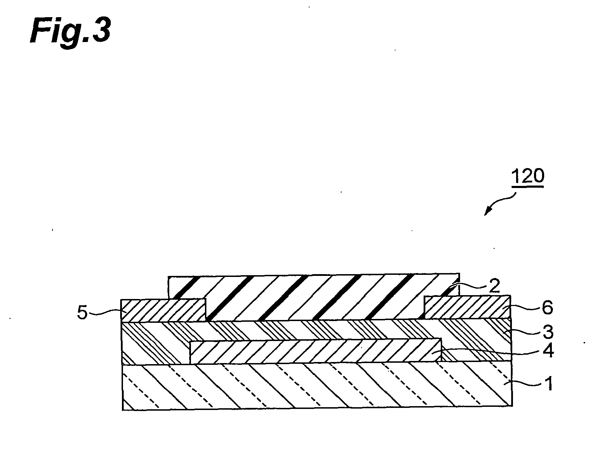

FIG 3 is a schematic cross-sectional diagram of an organic thin film transistor (field effect type organic thin film transistor) according to a third embodiment of the present invention. The organic thin film transistor 120 shown in FIG 3 is provided with a substrate 1; a gate electrode 4 formed on the substrate 1; an insulating layer 3 formed on the substrate 1, so as to cover the gate electrode 4; a source electrode 5 and a drain electrode 6 formed at a predetermined distance on the insulating layer 3, so as to partially cover a region of the insulating layer 3 with a gate electrode 4 formed thereunder; and an active layer 2 formed on top of the insulating layer 3, so as to partially cover the source electrode 5 and the drain electrode 6.

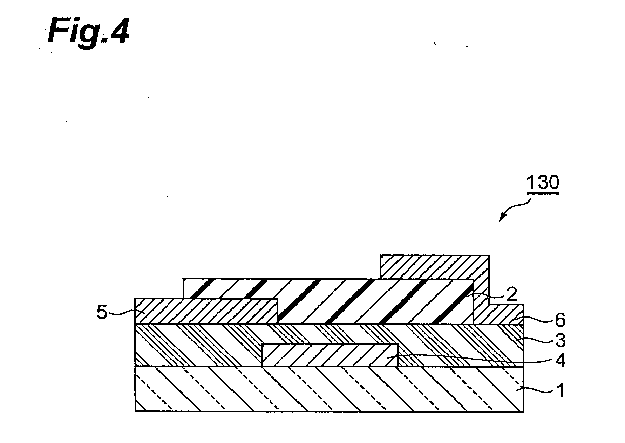

FIG. 4 is a schematic cross-sectional diagram of an organic thin film transistor (field effect type organic thin film transistor) according to a fourth embodiment of the present invention. The organic thin film transistor 130 shown in FIG 4 is provided with a substrate 1; a gate electrode 4 formed on the substrate 1; an insulating layer 3 formed on the substrate 1, so as to cover the gate electrode 4; a source electrode 5 formed on the insulating layer 3, so as to partially cover a region of the insulating layer 3 with a gate electrode 4 formed thereunder; an active layer 2 formed on top of the insulating layer 3, so as to partially cover the source electrode 5; a drain electrode 6 formed on the insulating layer at a predetermined distance from source electrode 5, so as to partially cover a region of the active layer 2 with a gate electrode 4 formed thereunder.

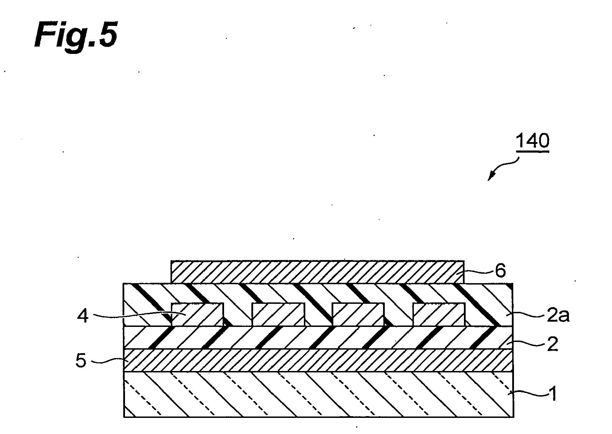

FIG. 5 is a schematic cross-sectional diagram of an organic thin film transistor (static induction type organic thin film transistor) according to a fifth embodiment of the present invention. The organic thin film transistor 140 shown in FIG 5 is provided with a substrate 1; a source electrode 5 formed on the substrate 1; an active layer 2 formed on the source electrode 5; a plurality of gate electrodes 4 formed at a predetermined distances on the active layer 2; an active layer 2a (the material constituting the active layer 2a may either be the same or different from that of the active layer 2) formed on the active layer 2, so as to cover the entire gate electrode 4; and a drain electrode 6 formed on the active layer 2a.

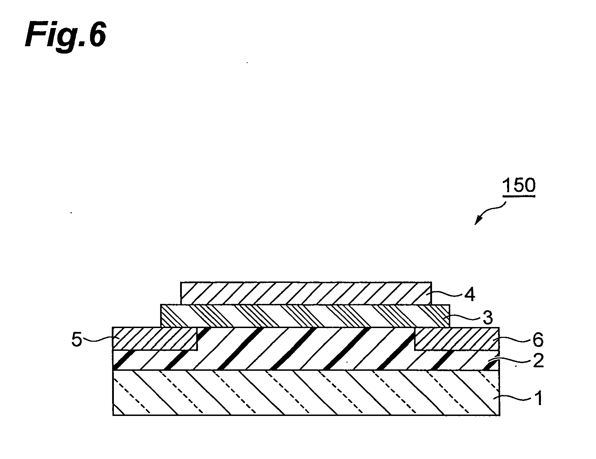

FIG 6 is a schematic cross-sectional diagram of an organic thin film transistor (field effect type organic thin film transistor) according to a sixth embodiment of the present invention. The organic thin film transistor 150 shown in FIG 6 is provided with a substrate 1; an active layer 2 formed on the substrate 1; a source electrode 5 and a drain electrode 6 formed at a predetermined distance on the active layer 2; an insulating layer 3 formed on the active layer 2, so as to partially cover the source electrode 5 and drain electrode 6; and a gate electrode 4 formed on the insulating layer 3, so as to partially cover a region of the insulation layer 3 with a source electrode 5 formed thereunder and a region of the insulation layer 3 with a drain electrode 6 formed thereunder, respectively.

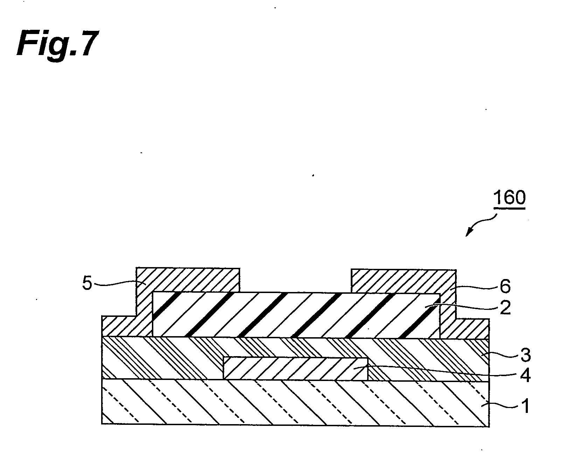

FIG. 7 is a schematic cross-sectional diagram of an organic thin film transistor (field effect type organic thin film transistor) according to a seventh embodiment of the present invention. The organic thin film transistor 160 shown in FIG 7 is provided with a substrate 1; a gate electrode 4 formed on the substrate 1; an insulating layer 3 formed on the substrate 1, so as to cover the gate electrode 4; an active layer 2 formed so as to cover a region of the insulating layer 3 with the gate electrode 4 formed thereunder; a source electrode 5 formed on the insulating layer 3, so as to partially cover a region of the active layer 2 with the gate electrode 4 formed thereunder; and a drain electrode 6 formed on the insulating layer 3 at a predetermined distance from the source electrode 5, so as to partially cover a region of the active layer 2 with the source electrode 5 formed thereunder.

In an organic thin film transistor according to a first to seventh embodiment, the active layer 2 and/or the active layer 2a contains the fluorine-containing compound of the present invention, and forms a current channel between the source electrode 5 and the drain electrode 6. Moreover, the gate electrode 4 controls the amount of current flowing through the current channel in the active layer 2 and/or the active layer 2a via the application of voltage.

A field effect type organic thin film transistor such as this may be manufactured by a known method, for example, the method described in Japanese Patent Application Laid-Open Publication No. H5-110069. Furthermore, the static induction type organic thin film transistor may be manufactured by a known method, for example, the method described in Japanese Patent Application Laid-Open Publication No. 2004-006476.

The substrate 1 is not particularly limited in any manner so long as it does not interfere with the characteristic features as an organic thin film transistor. Thus, a glass substrate, a flexible film substrate, or a plastic substrate may be employed.

Since the employment of an organic solvent-soluble compound is highly advantageous from a production aspect when forming the active layer 2, and thus preferred, the above-described production method of the organic thin film of the present invention may be employed to form an organic thin film formed with an active layer 2.

The insulating layer 3 in contact with active layer 2 is not particularly limited in any manner as long as the material is high in electrical insulating properties, and thus a known material may be employed. For example, SiOx, SiNx, Ta₂O₅, polyimide, polyvinyl alcohol, polvinylphenol, organic glass, and photoresist may be exemplified. From the standpoint of low voltage, a material with high conductivity is preferable.

In cases where the active layer 2 is formed on the insulating layer 3, the active layer 2 may also be formed after surface modification by treating a surface of the insulating layer 3 with a surface treating agent such as a silane coupling agent, in order to improve the interfacial properties between the insulating layer 3 and the active layer 2. As the surface treating agent, a long-chain alkylchlorosilane, a long-chain alkylalkoxysilane, a fluorinated alkylchlorosilane, a fluorinated alkylalkoxysilane, and a silylamine compound such as hexamethyldisilazane may be exemplified. It is also possible to pre-treat the surface of the insulating layer with ozone UV or O₂ plasma, prior to treatment with the surface treating agent.

After production of the organic thin film transistor, it is preferable that a protective film is formed on the organic thin film transistor, in order to protect the device. Accordingly, the organic thin film transistor may be blocked from air, and a reduction in the characteristic features of the organic thin film transistor may be prevented. Moreover, the affects from a step of forming an operating display device on the organic thin film transistor can be reduced via the protective film.

As a method of forming the protective film, a method of covering it with a UV cured resin, a thermosetting resin, or an inorganic SiONx film or the like, may be provided. It is preferable that the steps during which the organic thin film transistor is first produced up to that where the productive film is formed are conducted without exposure to air (for example, within a dry nitrogen atmosphere, or within a vacuum), in order to effectively block the organic thin film transistor from air.

Next, the applications of the organic thin film of the present invention to a solar cell will be described.

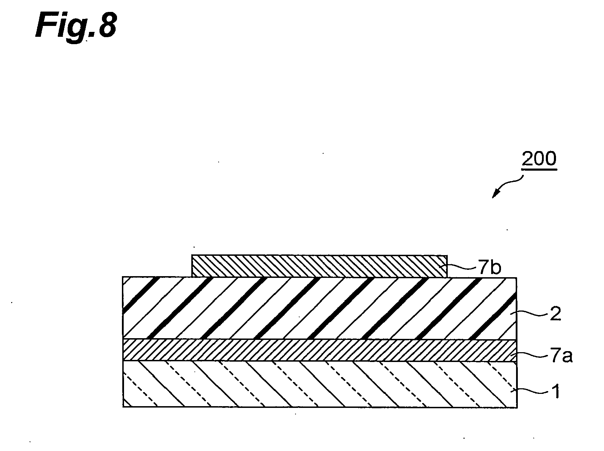

FIG 8 is a schematic cross-sectional diagram of a solar cell according to an embodiment. The solar cell 200 shown in FIG 8 is provided with a substrate 1; a first electrode 7a formed on the substrate 1; an active layer 2 composed of an organic thin film containing the fluorine-containing compound of the present invention and formed on the first electrode 7a; and a second electrode 7b formed on the active layer 2.

In the solar cell according to the present embodiment, a transparent or semi-transparent electrode is used for either the first electrode 7a or a second electrode 7b. As the electrode material, a metal such aluminum, gold, silver, copper, an alkali metal, and an alkaline earth metal; or a semi-transparent film, or transparent conductive film thereof may be employed. In order to obtain a high open-circuit voltage, it is preferable that each respective electrode is selected so as to have a large difference in work function. An electric charge generation material, a sensitizing agent, and the like, may also be added and employed, in order to increase light sensitivity in the active layer 2 (organic thin film). As the substrate 1, a silicon substrate, a glass substrate, a plastic substrate, and the like may be employed.

Next, the applications of the organic thin film of the present invention to a photosensor will be described.

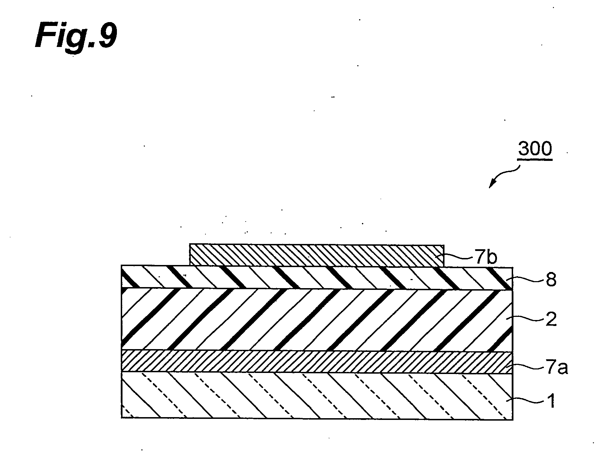

FIG 9 is a schematic cross-sectional diagram of a photosensor according to a first embodiment. The photosensor 300 shown in FIG 9 is provided with a substrate 1; a first electrode 7a formed on the substrate 1; an active layer 2 composed of an organic thin film containing the fluorine-containing compound of the present invention and formed on the first electrode 7a; a charge generation layer 8 formed on the active layer 2; and a second electrode 7b formed on the charge generation layer 8.

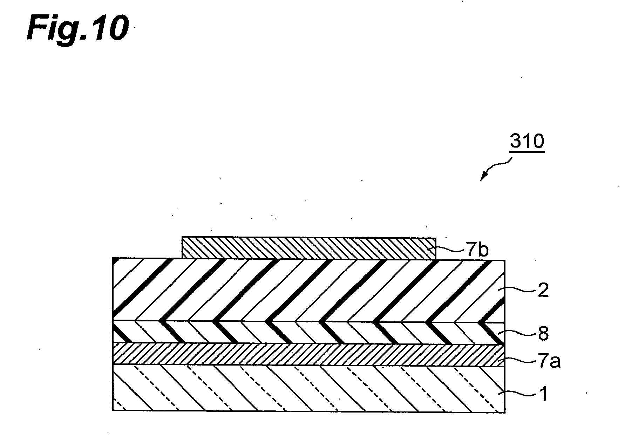

FIG. 10 is a schematic cross-sectional diagram of a photosensor according to a second embodiment. The photosensor 310 shown in FIG 10 is provided with a substrate 1; a first electrode 7a formed on the substrate 1; a charge generation layer 8 formed on the first electrode 7a; an active layer 2 composed of an organic thin film containing the fluorine-containing compound of the present invention and formed on the charge generation layer 8; and a second electrode 7b formed on the active layer 2.

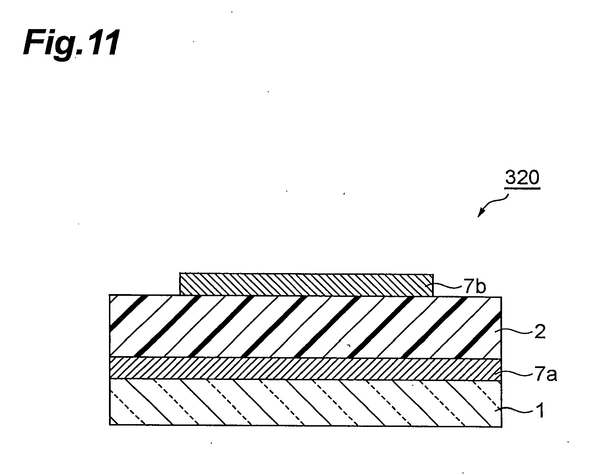

FIG. 11 is a schematic cross-sectional diagram of a photosensor according to a third embodiment. The photosensor 320 shown in FIG 11 is provided with a substrate 1; a first electrode 7a formed on the substrate 1; an active layer 2 composed of an organic thin film containing the fluorine-containing compound of the present invention and formed on the first electrode 7a; and a second electrode 7b formed on the active layer 2.

In a photosensor according to a first to third embodiment, a transparent or semi-transparent electrode is used for either the first electrode 7a or a second electrode 7b. The charge generation layer 8 is a layer generating an electric charge by the absorption of light. As the electrode material, a metal such aluminum, gold, silver, copper, an alkali metal, and an alkaline earth metal; or a semi-transparent film, or transparent conductive film thereof may be employed. An electric charge generation material, a sensitizing agent, and the like, may also be added and employed, in order to increase light sensitivity in the active layer 2 (organic thin film). Moreover, as the substrate 1, a silicon substrate, a glass substrate, a plastic substrate, and the like may be employed.

### Examples

Hereinafter, the present invention will be described in greater detail based on Examples and Comparative Examples. However, the present invention is not specifically limited to the below-mentioned Examples.

### (Measuring Conditions and the like)

Nuclear magnetic resonance spectra (NMR) were measured with a JMN-270 (270 MHz at the time of ¹H measurement), or a JMNLA-600 (600 MHz at the time of ¹⁹F measurement), both of which are the tradenames by JEOL, Ltd. Chemical shifts were represented as parts per million (ppm). Tetramethylsilane (TMS) was used as an internal standard (0 ppm). A binding constant (J) was indicated in Hz, and the letters s, d, t, q, m, and br, were each respectively used to represent, a singlet, a doublet, a triplet, a quartet, a multiplet, and broad. Moreover, mass spectrometry (MS) was performed using a GCMS-QP5050A (tradename) that was manufactured by Shimadzu Corp., via an electron ionization (EI) or direct inlet (DI) method. The Silicon gel employed in column chromatography separation was Silicagel 60N (40 µm to 50 µm), which is a tradename by Kanto Kagaku Co, Ltd. All of the chemical substances were reagent grade, and purchased from Wako Pure Chemical Industries, Ltd.; Tokyo Kasei Kogyo Co., Ltd.; Kanto Kagaku Co, Ltd; Nacalai Tesque, Inc.; Sigma Aldrich Japan, K.K.; or Daikin Chemicals Co., Ltd.

Cyclic voltammetry was measured using a tradename CV-50W by BAS Inc. as the measuring device, with a Pt electrode manufactured by BAS Inc. being the work electrode, a Pt wire being the counter electrode, and an Ag wire being the reference electrode. The sweep rate during measurement was 100 mV/sec, and the scanning potential region was -2.8 V to 1.6 V. The measurement of reduction potential and oxidation potential was performed after 1×10⁻³ mol/L of the compound and polymer, and 0.1 mol/L of tetrabutylammonium hexafluorophosphate (TBAPF6) as a supporting electrolyte were completely dissolved in a monofluorobenzene solvent.

### Reference Synthesis Example 1

### <Synthesis of compound A>

Indeno[1,2-b]fluorene-6-12-dione (2.23 g, 7.91 mmol), boron trifluoride-acetic acid complex (5.94 g, 31.6 mmol), ethanedithiol (2.98 g, 31.6 mmol), and chloroform (50 ml) were placed in a round-bottom flask and then reacted at 90°C. After 12 hours, water was added and the organic phase was extracted with chloroform. The organic phase was dried over magnesium sulfate and concentrated in vacuo. The solid matter was washed with ether, to obtain the target compound (3.41 g, 99% yield) as a white solid (compound A). TLC R_{f}=0.3 (hexane:chloroform = 2:1) : ¹H-NMR (270 MHz, CDCl₃): 87.90(s, 2H), 7.65(m, 4H), 7.33(m, 4H), 3.70(s, 8H): GC-MS(DI): m/z=434(M⁺).

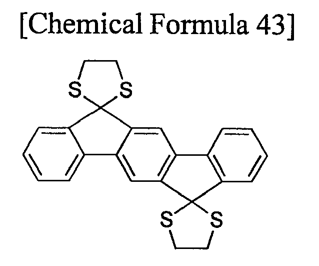

### Reference Synthesis Example 2

### <Synthesis of compound B>

After placing N-iodosuccinimide (6.00 g, 26.7 mmol) in a heat-dried three-necked flask and substituting with nitrogen, dichloromethane (50 ml) was added. The reaction solution was cooled to -78°C, and hydrogen fluoride-pyridine (10 ml) was added dropwise. After being agitated for 30 minutes at -78°C, a dichloromethane solution (50 ml) of compound A (1.50 g, 3.45 mmol) was added dropwise. After being agitated for 4 hours at -78°C until the temperature was warmed to room temperature, it was further agitated for 2 hours. After being concentrated in vacuo, purification was performed by silica gel column chromatography (hexane/choloroform), to obtain the target compound (1.01 g, 95% yield) as a white solid (compound B).
TLC R_{f}= 0.7 (hexane:chloroform = 2:1) : ¹H-NMR (270 MHz, CDCl₃): δ7.81(s, 2H), 7.63(t, 4H, J=8.2 Hz), 7.52(t, 2H, J=6.9 Hz), 7.41(t, 2H, J=7.3 Hz): GC-MS (EI): m/z=326 (M⁺).

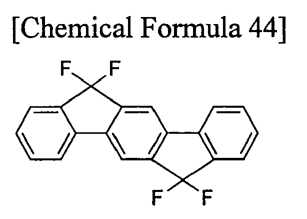

### Reference Synthesis Example 3

### <Synthesis of compound C>

After compound B (718 mg, 2.20 mmol) and carbon tetrachloride (40 ml) were placed in a round-bottom flask, [bis(trifluoroacetoxy)iodo]benzene (1.23 g, 4.87 mmol) and iodine (1.97 g, 4.78 mmol) were added and reacted at room temperature. After 17 hours, aqueous sodium thiosulfate was added, the organic phase was extracted with chloroform, and then the organic phase was dried with magnesium sulfate. After being concentrated in vacuo, the solid matter was washed with methanol, hexane, and acetone, to obtain the target compound (1.20 g, 94% yield) as a yellow-white solid (compound C).
TLC R_{f}=0.6 (hexane:chloroform = 4:1) : ¹H-NMR (270 MHz, CDCl₃): δ7.97(s, 2H), 7.85(d, 2H, J=8.0 Hz), 7.77(s, 2H), 7.34(d, 2H, J=8.0 Hz): GC-MS(EI): m/z=578(M⁺).

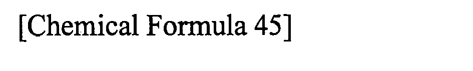

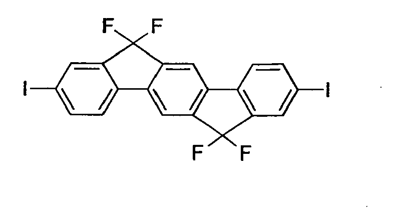

### Reference Synthesis Example 4

### <Synthesis of compound D>

Compound C (318 mg, 0.55 mmol) and tetrahydrofuran (26 ml) were placed in a heat-dried round-bottom flask. It was nitrogen substituted, cooled to -78°C, and then reacted by adding n-butyl lithium (1.6 M hexane solution, 0.80 mL, 1.43 mmol). After one hour, tributyltin chloride (500 mg, 1.54 mmol) was added at -78°C until the temperature was warmed to room temperature. After one hour, water was added and the organic phase was extracted with ethyl acetate. The organic phase was dried over magnesium sulfate and concentrated in vacuo. Then purification was performed by GPC (choloroform), to obtain the target compound (274 mg, 55% yield) as a pale yellow liquid (compound D).
TLC R_{f}=0.8 (hexane) : δ7.78(s, 2H), 7.73(s, 2H), 7.60(d, 2H, J=7.1 Hz), 7.54(d, 2H, J=7.1 Hz)), 1.33(m, 54H): MS(MALDI-TOF, 1,8,9-trihydroxyanthracene matrix) m/z=903.1(M⁺, Calcd 904.4).

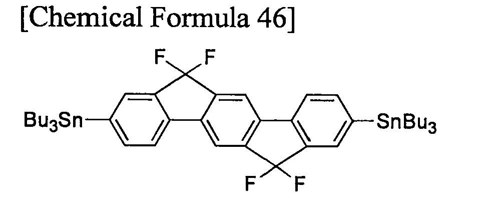

### Example 1

### <Synthesis of compound E>

After placing compound C (50 mg, 0.09 mmol), 4-fluorophenylboronic acid (38 mg, 0.27 mmol), tetrakis(triphenylphosphine)pallidium (0) (10 mg, 0.009 mmol), sodium carbonate (29 mg. 0.27 mmol), 1,2-dimethoxyethane (2 ml), and water (0.5 ml) were placed in a heat-dried stoppered test tube, it was substituted with nitrogen and reacted at 80°C. After 24 hours, water was added and the organic phase was extracted with chloroform. The organic phase was dried over magnesium sulfate and concentrated in vacuo. Purification was performed by silica gel column chromatography (choloroform), to obtain the target compound (32 mg, 37% yield) as a white solid (compound E).
TLC R_{f}=0.3 (hexane:chloroform) : ¹H-NMR (270 MHz, CDCl₃): δ8.22(m, 2H), 7.84(m, 2H), 7.69(s, 2H), 7.61(m, 2H): GC-MS(DI): m/z=514(M⁺).

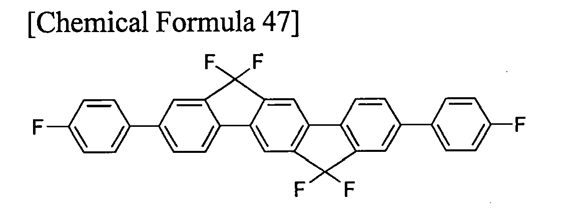

### Example 2

### <Synthesis of compound F>

After compound C (50 mg, 0.087 mmol), pentafluorophenylboronic acid (45 mg, 0.21 mmol), tetrakis(triphenylphosphine)pallidium (0) (7 mg, 0.006 mmol), silver oxide (I) (25 mg, 0.10 mmol), cesium fluoride (120 mg, 0.79 mmol), and 1,2-dimethoxyethane (3 ml) were added to a heat-dried stoppered test tube, it was substituted with nitrogen and reacted at 70°C. After 60 hours, water was added and the organic phase was extracted with chloroform. The organic phase was dried over magnesium sulfate and concentrated in vacuo. Purification was performed by silica gel column chromatography (hexane/choloroform), to obtain the target compound (35 mg, 62% yield) as a pale yellow solid (compound F).
TLC R_{f}=0.2 (chloroform) : ¹H-NMR (270 MHz, CDCl₃): δ7.89(s, 2H), 7.75(d, 2H, J=7.8 Hz), 7.73(s, 2H), 7.60(d, 2H, J=7.8 Hz): GC-MS(DI): m/z=658(M⁺).

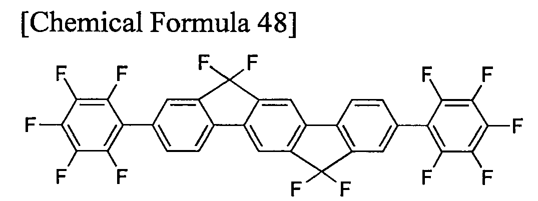

### Example 3

### <Synthesis of compound G>

After compound D (274 mg, 0.303 mmol), 4-acetylphenylboronic acid (306 mg, 1.21 mmol), tetrakis(triphenylphosphine)pallidium (0) (35 mg, 0.010 mmol), silver oxide (I) (25 mg, 0.10 mmol), and toluene (15 ml) were added to a heat-dried stoppered test tube, it was substituted with nitrogen and reacted at 120°C. After 42 hours, the solid matter that was filtered out was washed with methanol. Purification was conducted by sublimation, to obtain the target compound (139 mg, 60% yield) as a yellow solid (compound G).
¹H-NMR (400 MHz, CDCl₃): δ8.18(m, 4H), 7.83(m, 12H) : GC-MS(DI): m/z=670(M⁺).

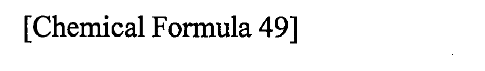

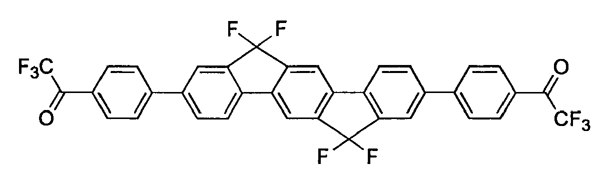

### Example 4

### <Preparation of organic thin film device A and evaluation of solar cell properties>

An o-dichlorobenzene solution was prepared using 2.0 wt % of compound E synthesized by Example 1 and poly(3-hexylthiophene) (P3HT, by Aldrich), respectively, and filtered with a 0.2 µm membrane filter. The solution of compound E and the solution of P3HT were compounded at volumetric ratio of 1:1, to thereby prepare a mixed coating solution. An organic thin film with a thickness of 70 nm was formed by spin coating using the abovementioned mixed coating solution onto a glass substrate having an ITO membrane with a thickness of 150 nm attached thereto by a sputter coating method. Approximately 4 nm of lithium fluoride, followed by 70 nm of aluminum, were deposited on the obtained organic thin film by a vacuum deposition method. Next, a glass substrate was adhered and sealed with a UV curable resin onto a surface thereof, and an organic thin film device A employing compound E and P3HT was prepared. Solar cell properties including a short-circuit current of 20 µA/cm² and an open-circuit voltage of 0.75 V were obtained after a voltage-current property was measured while irradiating AM 1.5 (100 mW/cm²) of artificial sunlight onto the obtained organic thin film device A with a solar simulator.

### Example 5

### <Evaluation of photosensor>

After a photo current during irradiation with 200 lx white light and a dark current during the absence of irradiation were measured using the organic thin film device A prepared by Example 4, a photo-to-dark-current ratio of 2.1×10² was obtained at an applied voltage -0.5 V, to thereby allow for the operation as photosensor thereof to be confirmed.

### Example 6

### <Preparation of organic thin film device B and evaluation of solar cell properties>

Similarly to Example 4, an organic thin film device B employing compound F and P3HT was prepared using the compound F synthesized by Example 2 in place of compound E. Solar cell properties including a short-circuit current of 35 µA/cm² and an open-circuit voltage of 1.08 V were obtained after a voltage-current property was measured while irradiating AM 1.5 (100 mW/cm²) of artificial sunlight onto the obtained organic thin film device B with a solar simulator.

### Example 7

### <Evaluation of photosensor>

After a photo current during irradiation with 200 lx white light and a dark current during the absence of irradiation were measured using the organic thin film device B prepared by Example 6, a photo-to-dark-current ratio of 1.1×10² was obtained at an applied voltage -0.5 V, to thereby allow for the operation as photosensor thereof to be confirmed.

### Example 8

### <Preparation of organic thin film device C and evaluation of solar cell properties>

Similarly to Example 4, an organic thin film device C employing the compound G and P3HT was prepared using the compound G synthesized by Example 3 in place of compound E. Solar cell properties including a short-circuit current of 64 µA/cm² and an open-circuit voltage of 1.08 V were obtained after a voltage-current property was measured while irradiating AM 1.5 (100 mW/cm²) of artificial sunlight onto the obtained organic thin film device C with a solar simulator.

### Example 9

### <Evaluation of photosensor>

After a photo current during irradiation with 200 1x white light and a dark current during the absence of irradiation were measured using the organic thin film device C prepared by Example 8, a photo-to-dark-current ratio of 2.3×10² was obtained at an applied voltage -0.5 V, to thereby allow for the operation as photosensor thereof to be confirmed.

### Example 10

### <Preparation of organic thin film device D and evaluation of transistor properties>

A substrate was prepared by forming silicon oxide film as an insulating layer on the surface of a highly doped n-type silicon wafer as a gate electrode via thermal oxidation, immersing it in hexamethyldisilazane (HMDS) at 50°C, and treating the silicon oxide film surface. Next, an organic thin film of compound G was accumulated on this surface treated substrate by a vacuum deposition method. Then, Au was vapor-deposited through a shadow mask onto this organic thin film, a source electrode and drain electrode were formed, and an organic thin film device D was prepared. Measuring the transistor properties in the obtained organic thin film device D by varying a gate voltage V_{G} and a source-drain voltage V_{SD} in a vacuum allowed for favorable Id-Vg properties to be obtained.

### Industrial Applicability

A fluorine-containing polycyclic aromatic compound, a fluorine-containing polymer, an organic thin film and organic thin film device of the present invention may be used in an organic thin film transistor, an organic solar cell, a photosensor, and the like.

## Claims

1. A fluorine-containing polycyclic aromatic compound represented by the following formula (I): 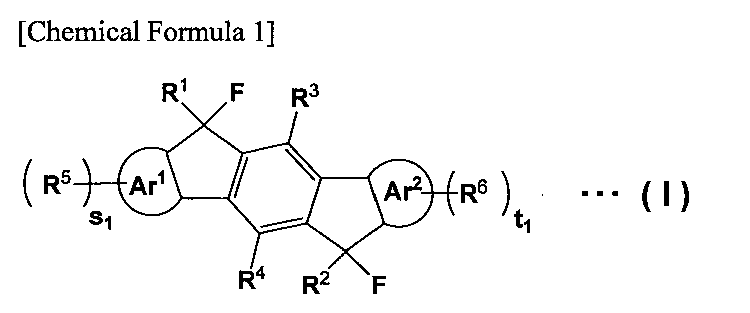 wherein in formula (I),
Ar¹ and Ar² each independently represent an aromatic hydrocarbon group having at least six carbon atoms or a heterocyclic group having at least four carbon atoms;
R¹, R², R³ and R⁴ each independently represent a hydrogen atom, a halogen atom, or a monovalent group;
R⁵ and R⁶ each independently represent a hydrogen atom or a monovalent organic group that may be substituted with a substituent, wherein the monovalent organic group is selected from among an alkyl group, an alkoxy group, an acyl group, an aryl group, an aryloxy group, an arylalkyl group, an arylalkoxy group and a heterocyclic group, and at least one of all the hydrogen atoms of the monovalent organic group including the substituent may be substituted with a fluorine atom;
s₁ and t₁ each independently represent an integer of at least 2;
with the proviso that
at least one of R⁵ and R⁶ is the monovalent organic group, in which at least one of all the hydrogen atoms of the monovalent organic group including the substituent is substituted with a fluorine atom; and
the plurality of R⁵ may be the same as or different from each other, and the plurality of R⁶ may be the same as or different from each other.

2. The fluorine-containing polycyclic aromatic compound according to claim 1, wherein the compound represented by the formula (I) is a compound represented by the following formula (II): 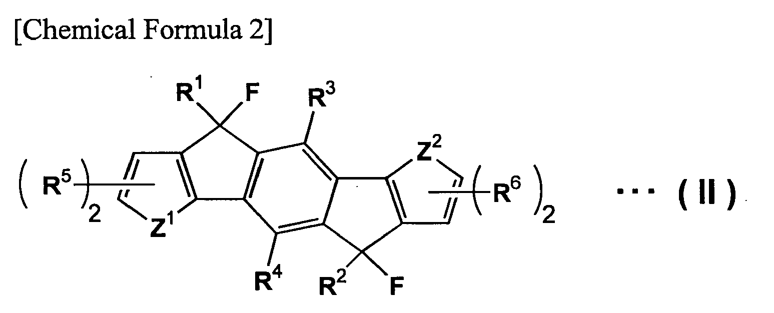 wherein in formula (II),
R¹, R², R³, R⁴, R⁵, and R⁶ are the same as above in meaning; and
Z¹ and Z² each independently represent any one of groups represented by the following formulae (i) to (ix); with the proviso that
at least one of R⁵ and R⁶ is the monovalent organic group, in which at least one of all the hydrogen atoms of the monovalent organic group including the substituent is substituted with a fluorine atom;
R⁷, R⁸, R⁹ and R¹⁰ each independently represent a hydrogen atom, a halogen atom, or a monovalent group, wherein R⁷ and R⁸ may be linked to each other to form a ring; and
the group represented by the formula (ii) may be reversed from left to right.

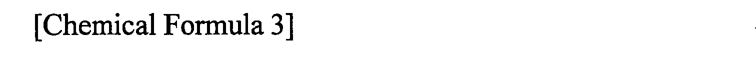

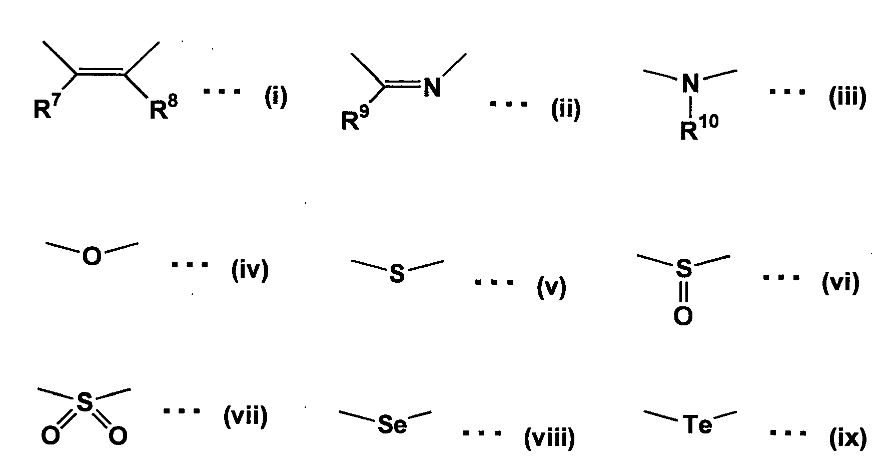

3. The fluorine-containing polycyclic aromatic compound according to claim 2, wherein the Z¹ and the Z² each are the group represented by the formula (i).

4. The fluorine-containing polycyclic aromatic compound according to any one of claims 1 to 3, wherein the R¹ and the R² each are a fluorine atom.

5. A fluorine-containing polymer having a repeating unit represented by the following formula (III): 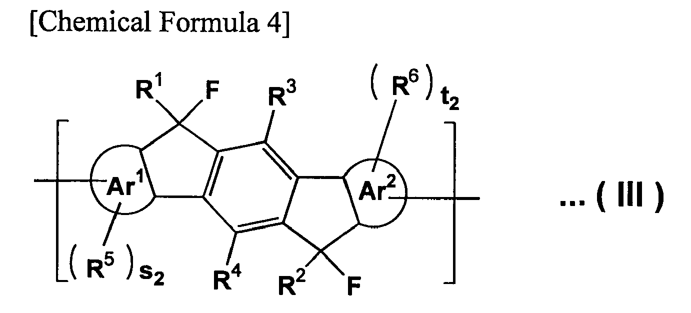 wherein in formula (III),
Ar¹ and Ar² each independently represent an aromatic hydrocarbon group having at least six carbon atoms or a heterocyclic group having at least four carbon atoms;
R¹, R², R³ and R⁴ each independently represent a hydrogen atom, a halogen atom, or a monovalent group;
R⁵ and R⁶ each independently represent a hydrogen atom or a monovalent organic group that may be substituted with a substituent, wherein the monovalent organic group is selected from among an alkyl group, an alkoxy group, an acyl group, an aryl group, an aryloxy group, an arylalkyl group, an arylalkoxy group and a heterocyclic group, and at least one of all the hydrogen atoms of the monovalent organic group including the substituent may be substituted with a fluorine atom;
s₂ and t₂ each independently represent an integer of at least 1;
the plurality of R⁵ may be the same as or different from each other when s₂ is at least 2, and the plurality of R⁶ may be the same as or different from each other when t₂ is at least 2.

6. The fluorine-containing polymer according to claim 5, wherein the repeating unit represented by the formula (III) is a repeating unit represented by the following formula (IV): 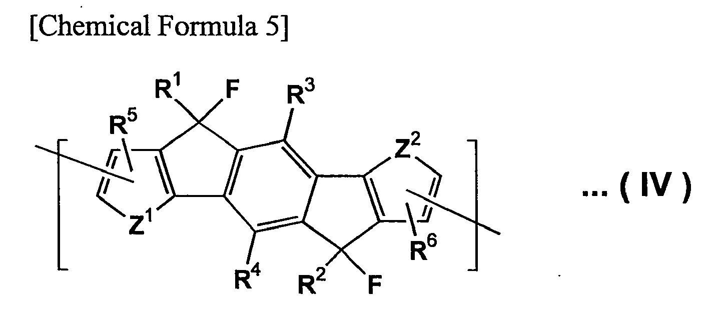 wherein in formula (IV),
R¹, R², R³, R⁴, R⁵, and R⁶ are the same as above in meaning;
Z¹ and Z² each independently represent any one of groups represented by the following formulae (i) to (ix);
R⁷, R⁸, R⁹ and R¹⁰ each independently represent a hydrogen atom, a halogen atom, or a monovalent group, wherein R⁷ and R⁸ may be linked to each other to form a ring; and
the group represented by the formula (ii) may be reversed from left to right.

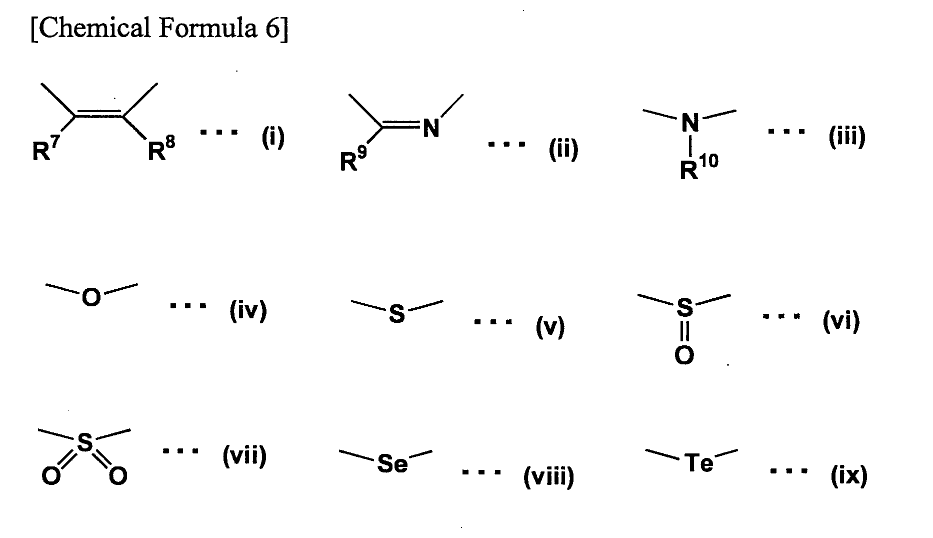

7. The fluorine-containing polymer according to claim 6, wherein the Z¹ and the Z² each are the group represented by the formula (i).

8. The fluorine-containing polymer according to any one of claims 5 to 7, having at least one repeating unit represented by the formula (III) and at least one repeating unit represented by the following formula (V): 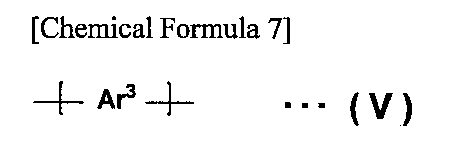 wherein in formula (V),
Ar³ represents a divalent aromatic hydrocarbon group or a divalent heterocyclic group, wherein these groups may be substituted with a substituent.

9. The fluorine-containing polymer according to claim 8, wherein the Ar³ is a group represented by the following formula (VI): 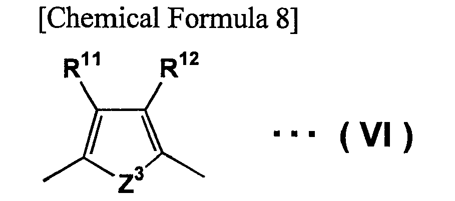 wherein in formula (VI),
R¹¹ and R¹² each independently represent a hydrogen atom, a halogen atom, or a monovalent group, wherein R¹¹ and R¹² may be linked to each other to form a ring;
Z³ represents any one of groups represented by the following formulae (i) to (ix);
wherein R⁷, R⁸, R⁹, and R¹⁰ each independently represent a hydrogen atom, a halogen atom, or a monovalent group, wherein R⁷ and R⁸ may be linked to each other to form a ring; and
the group represented by the formula (ii) may be reversed from left to right.

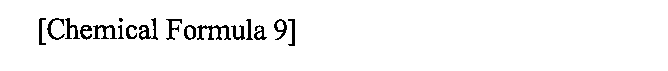

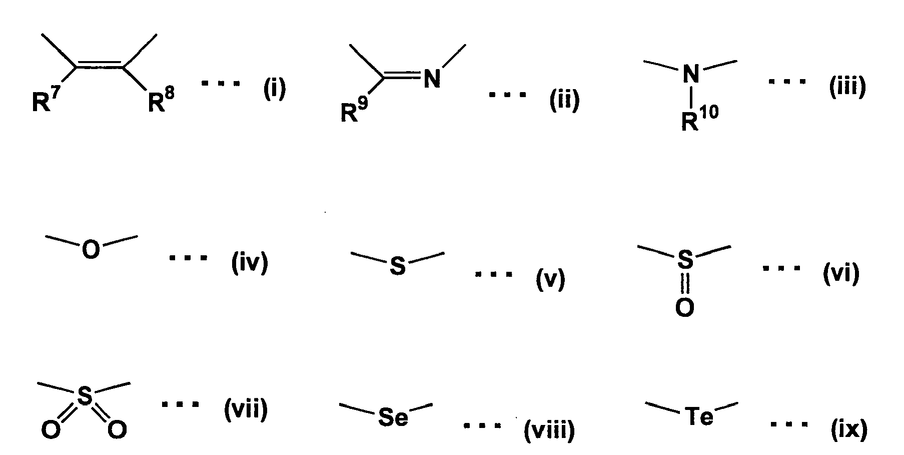

10. The fluorine-containing polymer according to claim 9, wherein the Z³ is the group represented by the formula (v).

11. The fluorine-containing polymer according to any one of claims 5 to 10, wherein the R¹ and the R² each are a fluorine atom.

12. An organic thin film comprising a fluorine-containing polycyclic aromatic compound according to any one of claims 1 to 4, and/or a fluorine-containing polymer according to any one of claims 5 to 11.

13. The organic thin film according to claim 12, wherein the organic thin film is formed by a vacuum deposition method, a spin coating method, inkjet printing method, a dispenser printing method, a flexographic printing method, a nozzle coating method, or a capillary coating method.

14. An organic thin film device comprising the organic thin film according to claim 12 or 13.

15. An organic thin film transistor, comprising:
a source electrode and a drain electrode;
an organic semiconductor layer forming a current channel therebetween; and
a gate electrode controlling an amount of current passing through the current channel; wherein
the organic semiconductor layer comprises an organic thin film according to claim 12 or 13.

16. An organic solar cell comprising the organic thin film according to claim 12 or 13.

17. A photosensor comprising the organic thin film according to claim 12 or 13.
